# EUROPEAN PATENT SPECIFICATION

(11) **EP 4 098 207 B1**
(45) Date of publication and mention of the grant of the patent: **12.08.2026**
(21) Application number: 21764329.5
(22) Date of filing: 29.01.2021
(51) Int. Cl.: A61B 17/00, A61F 2/95, A61F 2/24

(54) **HANDLE FOR CONVEYING IMPLANT, CATHETER ASSEMBLY, AND CONVEYING SYSTEM**
HANDGRIFF ZUM FÖRDERN VON IMPLANTATEN, KATHETERANORDNUNG UND FÖRDERSYSTEM
POIGNÉE POUR TRANSPORTER UN IMPLANT, ENSEMBLE CATHÉTER ET SYSTÈME DE TRANSPORT

(30) Priority: 06.03.2020 CN 202010149845; 06.03.2020 CN 202010149838
(43) Date of publication of application: 07.12.2022
(73) Proprietor: Shanghai Microport Cardioflow Medtech Co., Ltd., Shanghai 201203 (CN)
(72) Inventor: CHENG, Xiaoming, Shanghai 201203 (CN); LIU, Shihong, Shanghai 201203 (CN); CHEN, Guoming, Shanghai 201203 (CN); LI, Yu, Shanghai 201203 (CN); ZHAO, Jing, Shanghai 201203 (CN)
(74) Representative: Marks & Clerk LLP
(86) International application number: PCT/CN2021/074504
(87) International publication number: WO 2021/175056

(56) References cited:
- EP-B1- 2 898 857
- WO-A1-2018/161359
- CN-A- 104 173 121
- CN-A- 106 175 985
- CN-U- 201 481 290
- CN-U- 209 761 215
- CN-U- 209 984 374
- CN-U- 211 985 521
- CN-U- 212 165 822
- US-A1- 2011 213 450
- US-A1- 2018 110 621

## Description

### TECHNICAL FIELD

The present application relates to the technical field of medical devices, in particular to a handle, a catheter assembly, and a delivery system for delivering implants.

### BACKGROUND

According to the survey, the probability of middle-aged and elderly people suffering from coronary heart disease, cardiovascular and cerebrovascular disease, heart valve disease, tumor and other diseases is increasing year by year. These diseases directly affect the quality of life and even life safety of middle-aged and elderly people. Conventional surgical operations are still the first choice for seriously ill patients, but for patients with advanced age, multi-organ disease, history of thoracotomy, and poor physical recovery, the conventional surgical operations have high risks and high mortality. Some patients don't even have the chance to undergo surgery. In the past ten years, the international interventional therapy has made remarkable progress through continuous exploration, and has become the most promising branch in the field of interventional therapy.

The interventional therapy is a brand-new treatment technology developed by the international community in recent years, and has a principle of using modern high-tech means to carry out a kind of micro-trauma treatment, in which under the guidance of medical imaging device, special precision instruments are introduced into the human body to diagnose and locally treat lesions in the body. This technology has the characteristics of no operation, small trauma, quick recovery, good effect and the like, which avoids the harm caused by the conventional surgical operations to patients.

As a power source during the entire interventional therapy, a handle usually includes a pure manual handle, a pure electric handle or a manual-electric hybrid handle, which must ensure sufficient safety, effectiveness, and economy. On the one hand, the handle has the risk of failure during the operation. If the current handle has a safety failure, the only option is to urgently convert the interventional operation to the surgical operation, which will bring greater physical trauma to the patient and even threaten life. On the other hand, the handle includes many parts, and currently the handles on the market cannot be reused, so the cost is higher, the corresponding operation cost is higher, and the patient's economic burden is greater. US2018/110621 A1 discloses a handle with a detachable connection with the catheter assembly.

### SUMMARY

The invention discloses a handle, a catheter assembly and a system as defined in claims 1-18.

The technical problem to be solved by the present application is to provide a handle, a catheter assembly, and a delivery system for delivering implants. When the handle fails during operations, the handle can be quickly replaced to avoid surgical risks, while the utilization rate of the handle can be improved, and the costs can be reduced.

The technical solution adopted by the present application to solve the above-mentioned technical problems is to provide a handle for delivering implants, which includes a handle housing, a driving device, and a transmission component. The handle housing is provided with a hollow first slot. A first connector is provided on the handle housing. The first connector is configured to be detachably connected to a catheter assembly. The driving device is connected to the transmission component, and controls the transmission component to move. The transmission component is provided in the handle housing, and includes a driving member. The driving member is moved towards a proximal end or distal end of the handle under a control of the driving device.

Preferably, the driving device includes a switch, a power supply, a control board, and a motor. The switch is connected to the power supply. The power supply is connected to the control board. The control board is connected to the motor. The motor includes an output end. The control board controls the output end of the motor to rotate.

Preferably, the transmission component includes a first large gear, a first pinion gear, and a screw rod. The first large gear is coaxial with and fixedly connected to the output end of the motor. The first large gear is meshed with the first pinion gear. The first pinion gear is coaxial with the screw rod, and fixedly connected to one end of the screw rod. A fixing component is matched and connected to the screw rod. The driving member is fixed on the fixing component.

Preferably, the transmission component includes a screw rod and a coupling. The screw rod is coaxial with the output end of the motor. One end of the coupling is fixedly connected to the output end of the motor, another end of the coupling is fixedly connected to one end of the screw rod. A fixing component is matched and connected to the screw rod; and the driving member is fixed on the fixing component.

Preferably, the driving device includes a manual rotating member and a gear shaft. The transmission component includes a second large gear and a second pinion gear. The manual rotating member includes a manual control member and a rotating shaft that are fixedly connected. The manual control member is arranged outside the handle housing. The rotating shaft passes through the handle housing, and is coaxial with and fixedly connected to the gear shaft. The gear shaft is coaxial with and fixedly connected to the second large gear. The second large gear is meshed with the second pinion gear. The second pinion gear is coaxial with the screw rod; and the second pinion gear is fixedly connected to another end of the screw rod.

Preferably, the driving device includes a manual rotating member and a gear shaft. The manual rotating member includes a manual control member and a rotating shaft that are fixedly connected. The manual control member is arranged outside the handle housing. The rotating shaft passes through the handle housing, and is coaxial with and fixedly connected to the gear shaft.

Preferably, the transmission component includes a second large gear, a second pinion gear, and a screw rod. The second large gear is coaxial with and fixedly connected to the gear shaft. The second large gear is meshed with the second pinion gear. The second pinion gear is coaxial with the screw rod, and is fixedly connected to an end of the screw rod. A fixing component is matched and connected to the screw rod; and the driving member is fixed on the fixing component.

Preferably, the driving device includes a switch, a power supply, a control board, and a motor. The switch is connected to the power supply. The power supply is connected to the control board. The control board is connected to the motor. The motor has a motor shaft. The control board controls the motor shaft to reciprocate in an axial direction.

Preferably, the driving member is fixed on the motor shaft.

Another technical solution adopted by the present application to solve the above-mentioned technical problems is to provide a catheter assembly for delivering implants, which includes a catheter assembly housing, an inner tube, an outer tube, and an outer tube base. A second connector is provided on the catheter assembly housing. The second connector is configured to be detachably connected to the handle as described above. A hollow second slot is provided on the catheter assembly housing. The inner tube passes through the outer tube base and is located in the outer tube. The outer tube base is movably sleeved outside the inner tube; and a distal end of the outer tube base is fixedly connected to a proximal end of the outer tube.

Preferably, the catheter assembly further includes an inner tube evacuation tube. A proximal end of the inner tube evacuation tube is provided on a housing at a proximal end of the catheter assembly housing. A distal end of the inner tube evacuation tube is fixedly connected to a proximal end of the inner tube.

Preferably, an outer tube evacuation tube communicating with the outer tube is provided on the outer tube base; and the outer tube evacuation tube extends out of the catheter assembly housing.

Preferably, the catheter assembly further includes a stabilizing tube base and a stabilizing tube. The stabilizing tube base is fixed on a distal end of the catheter assembly housing. A proximal end of the stabilizing tube is fixedly connected to a distal end of the stabilizing tube base. A distal end of the outer tube passes through the stabilizing tube base and the stabilizing tube.

Preferably, a stabilizing tube evacuation tube communicating with the stabilizing tube is provided on the stabilizing tube base; and the stabilizing tube evacuation tube extends out of and is exposed to the catheter assembly housing.

Another technical solution adopted by the present application to solve the above-mentioned technical problems is to provide a delivering system for delivering implants, which includes the handle as describled above and the catheter assembly as describled above. The second connector is detachably connected to the first connector. A position and a shape of the second slot correspond to a position and a shape of the first slot on the handle housing. The outer tube base is fixedly connected to the driving member in an axial direction, and the outer tube base is detachably connected to the driving member in a third direction.

Preferably, a first groove is provided on the driving member, a second groove is provided on the outer tube base. The first groove cooperates with the second groove and is sleeved inside the second groove.

Preferably, the detachable connection of the first connector and the second connector is a slot connection, a threaded connection, a snap connection, a pin connection or a spline connection.

Preferably, the connection between the first connector and the second connector is a slot connection. The first connector has a detaching button and a third slot. The second connector is a protrusion having a limiting component in a third direction. A first through hole and a second through hole are provided on the handle housing. A proximal end of the first connector passes through the first through hole so that the third slot is located at an inner side of the handle housing, and the detaching button is located at an outer side of the handle housing. The handle housing has a locking position and a detaching position. The detaching button is capable of being toggled between the locking position and the detaching position. After passing through the second through hole, the protrusion is located at the inner side of the handle housing; when the protrusion toggles the detaching button to the locking position, the third slot is engaged with the protrusion.

Another technical solution adopted by the present application to solve the above-mentioned technical problems is to provide a handle for delivering implants, which includes a handle housing, a driving device, and a transmission component. The handle housing is provided with a hollow first slot. A first engaging component is provided on the handle housing in a first direction. The first engaging component is configured to be detachably connected to the catheter assembly. The driving device is connected to the transmission component, and controls the transmission component to move. The transmission component is provided in the handle housing, and includes a driving member. The driving member is moved towards a proximal end or distal end of the handle in the first slot under a control of the driving device.

Preferably, the first engaging component comprises a first engaging slot and a first engaging strip. The first engaging strip protrudes in a second direction and is arranged in the first engaging slot.

Preferably, the first engaging slot extends in a first direction to form a first guiding rail.

Preferably, the first engaging component comprises a third protruding portion and a fourth protruding portion that are connected to each other. The third protruding portion extends in a first direction, and the fourth protruding portion extends in a third direction. A second opening is formed between the third protruding portion, the fourth protruding portion and an edge of the handle housing; and the second opening faces the distal end of the handle.

Preferably, a fastening component is provided on a side of the handle housing.

Preferably, the fastening component is a threaded knob. The threaded knob includes a threaded portion and a knob portion. The knob portion is provided at an outer side of a distal end of the handle housing, and extends out of an edge of the handle housing in a third direction. The threaded portion passes through the handle housing and is threadedly connected to a fixing member provided in the handle housing.

Preferably, the driving device inlcudes a switch, a power supply, a control board and a motor. The switch is connected to the power supply. The power supply is connected to the control board. The control board is connected to the motor. The motor has an output end. The control board controls the output end of the motor to rotate.

Preferably, the transmission component includes a screw rod and a coupling. The screw rod is coaxial with the output end of the motor. One end of the coupling is fixedly connected to the output end of the motor, another end of the coupling is fixedly connected to one end of the screw rod. A fixing component is matched and connected to the screw rod; and the driving member is fixed on the fixing component.

Preferably, the driving device includes a manual rotating member and a gear shaft. The transmission component includes a large gear and a pinion gear. The manual rotating member includes a manual control member and a rotating shaft that are fixedly connected. The manual control member is arranged at an outer side of a proximal end of the handle housing. The rotating shaft passes through the handle housing, and is coaxial with and fixedly connected to the gear shaft. The gear shaft is coaxial and fixedly connected to the large gear. The large gear is meshed with the pinion gear. The pinion gear is coaxial with the screw rod, and the pinion gear is fixedly connected to another end of the screw rod.

Another technical solution adopted by the present application to solve the above-mentioned technical problems is to provide a catheter assembly for delivering implants, which includes a catheter assembly housing, an inner tube, an outer tube and an outer tube base. A hollow second slot is provided on the catheter assembly housing. A second engaging component is provided on the catheter assembly housing in a first direction. The second engaging component is configured to be detachably connected to the handle as described above. The inner tube passes through the outer tube base and is located in the outer tube. The outer tube base is movably sleeved outside the inner tube, and is moved towards a proximal end or a distal end of the catheter assembly in the second slot. A distal end of the outer tube base is fixedly connected to a proximal end of the outer tube.

Preferably, the second engaging component includes a second engaging slot and a second engaging strip. The second engaging strip protrudes in a second direction and is arranged in the second engaging slot.

Preferably, the second engaging slot extends in the first direction to form a second guiding rail.

Another technical solution adopted by the present application to solve the above-mentioned technical problems is to provide a delivering system for delivering implants, which includes the handle as described above and the catheter assembly as described above. A position and a shape of the second slot correspond to a position and a shape of the first slot on the handle housing. A position and a shape of the first engaging component correspond to a position and a shape of the second engaging component. The handle is detachably connected to the catheter assembly in a first direction through the first engaging component and the second engaging component. The outer tube base is fixedly connected to the driving member in an axial direction, and the outer tube base is detachably connected to the driving member in a third direction.

Preferably, a first groove is provided on the driving member, a second groove is provided on the outer tube base. The first groove cooperates with the second groove and is sleeved inside the second groove.

Preferably, the first engaging component includes a first engaging slot and a first engaging strip. The first engaging strip protrudes in a second direction and is arranged in the first engaging slot. The second engaging component includes a first protruding portion and a second protruding portion that are connected to each other. A first opening is formed between the first protruding portion, the second protruding portion and an edge of the catheter assembly housing. The first opening faces a proximal end of the catheter assembly and is engaged with the first engaging strip.

Preferably, a threaded knob is provided on a side of the handle housing. The threaded knob includes a threaded portion and a knob portion. The knob portion is provided at an outer side of a distal end of the handle housing. A surface of a proximal end of the knob portion extends out of an edge of the handle housing in a third direction, to be abutted against an outer side of a distal end of the catheter assembly housing.

Compared with the prior art, the present application has the following beneficial effects. The handle, the catheter assembly and the delivery system for delivering implants according to the present application adopt a detachable connection between the handle and the catheter assembly; and the present application also skillfully utilizes the feature that the outer tube assembly and the inner tube assembly can have relative axial displacement, combines the detachable connection between the driving member and the outer tube assembly or the inner tube assembly in the third direction, realizing that the handle can be detached from the catheter assembly after the occurrence of the displacement between the handle and the catheter assembly in the first direction. The connecting process is simple and time-saving. During an operation, if the handle is failed, the handle can be quickly disassembled and replaced. Therefore, the present application can quickly deal with the safety risk of handle failure and avoid more trauma to the patient caused by the change from the interventional surgery to the surgical operation due to the handle problem. In addition, the separate design of the handle and the catheter assembly enables the handle to be reusable, reduces the cost of each operation, and reduces the economic burden of the patient. The catheter assembly and the handle are individually integrally formed, so that the operation of the handle and the evacuating operation of the catheter assembly do not interfere with each other.

### BRIEF DESCRIPTION OF THE DRAWINGS

Accompanying drawings are used for better understanding of the present application and do not constitute an improper limitation of the present application, in which:
FIG. 1 is a schematic view showing an overall structure of a delivery system according to a first embodiment of the present application;
FIG. 2 is a schematic view showing an internal structure of the delivery system according to the first embodiment of the present application;
FIG. 3 is a schematic view showing an internal structure of a handle according to the first embodiment of the present application;
FIG. 4 is a schematic view showing an internal structure of a catheter assembly according to the first embodiment of the present application;
FIG. 5 is a partial exploded view of a detachable connecting component of the delivery system according to the first embodiment of the present application;
FIG. 6 is a structural schematic view of a driving member according to the first embodiment of the present application;
FIG. 7 is a schematic view showing an overall structure of the delivery system according to the first embodiment of the present application;
FIG. 8 is a partial enlarged view of an inner tube assembly according to the first embodiment and a second embodiment of the present application;
FIG. 9 is a schematic view showing an overall structure of a delivery system according to the second embodiment of the present application;
FIG. 10 is a schematic view showing an internal structure of the delivery system according to the second embodiment of the present application;
FIG. 11 is a schematic view showing an internal structure of a handle according to the second embodiment of the present application;
FIG. 12 is a schematic view showing an internal structure of a catheter assembly according to the second embodiment of the present application;
FIG. 13 is a partial schematic view of a handle housing according to the second embodiment of the present application;
FIG. 14 is a structural schematic view of a driving member according to the second embodiment of the present application; and
FIG. 15 is a schematic view showing an overall structure of the delivery system according to the second embodiment of the present application.

In figures:
1- handle; 2- catheter assembly; 101- first housing; 102- second housing; 103-switch; 104- power supply; 105- control board; 106- motor; 107- first large gear; 108- first pinion gear; 109- first connector; 110- screw rod; 111- driving member; 112- manual rotating member; 113- gear shaft; 114- first slot; 115- second large gear; 116- second pinion gear; 117- control key; 118- first engaging component; 119- fastening component; 120-coupling; 121- pinion gear; 122- large gear; 123- a fixing member; 201- third housing; 202- fourth housing; 203- stabilizing tube base; 204- stabilizing tube; 205- outer tube; 206-inner tube assembly; 207- stabilizing tube evacuation tube; 208- outer tube evacuation tube; 209- outer tube base; 210- inner tube evacuation tube; 211- second slot; 1011- first through hole; 1012- second through hole; 1021- first engaging strip; 1022- third engaging strip; 1023- first engaging slot; 1024- first guiding rail; 2011- protrusion; 1092- detaching button; 1091- third slot; 1111- first groove; 1121- manual control member; 1122- rotating shaft; 1061- output end; 1191- knob portion; 1192- threaded portion; 2012- second engaging component; 2012a- first protruding portion; 2012b- second protruding portion; 2012c- first opening; 2091- second groove; 2061- conical head; 2062- fixed head; 2063- inner tube; 3-implant; 10- handle housing; 20- catheter assembly housing.

### DETAILED DESCRIPTION OF THE EMBODIMENTS

The present application will be further described below with reference to the accompanying drawings and embodiments.

It should be noted that the terms "inner", "outer", "upper", "lower" and similar expressions used in the present application are only for the purpose of illustration and do not represent the only embodiment. In the present application, terms "distal end", "proximal end", "axial direction", "first direction", "second direction", and "third direction" are used, where the "distal end" is a side far away from an operator of a delivery system; the "proximal end" is a side close to the operator of the delivery system; the "axial direction" refers to an axial direction of an outer tube, which is the "first direction"; the "second direction" refers to a direction perpendicular to an axis of the outer tube on a horizontal plane where the axis of the outer tube is located; the "third direction" refers to a direction perpendicular to the axis of the outer tube on a vertical plane where the axis of the outer tube is located.

The present application provides a detachable handle. If the handle fails during operation, the failed handle can be quickly disassembled and replaced, and then the operation can be continued without affecting the operation process. Moreover, after the operation, the detachable handle can be disassembled separately for sterilization and reuse, so that the cost of the handle can be allocated to multiple operations, thereby reducing the cost of the handle in one operation and reducing the economic burden of the patient.

### First Embodiment

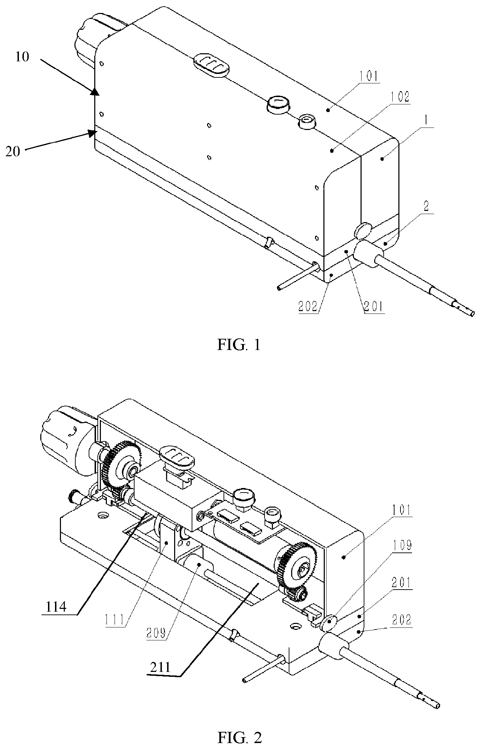

Referring to FIG. 1, a delivery system for delivering implants according to this embodiment includes a handle 1 and a catheter assembly 2. The catheter assembly 2 is located outside the handle 1. The catheter assembly 2 and the handle 1 are detachably connected. In order to facilitate assembly, preferably, the handle 1 includes a first housing 101 and a second housing 102. The first housing 101 and the second housing 102 are fixedly connected. The catheter assembly 2 includes a third housing 201 and a fourth housing 202. The third housing 201 and the fourth housing 202 are fixedly connected.

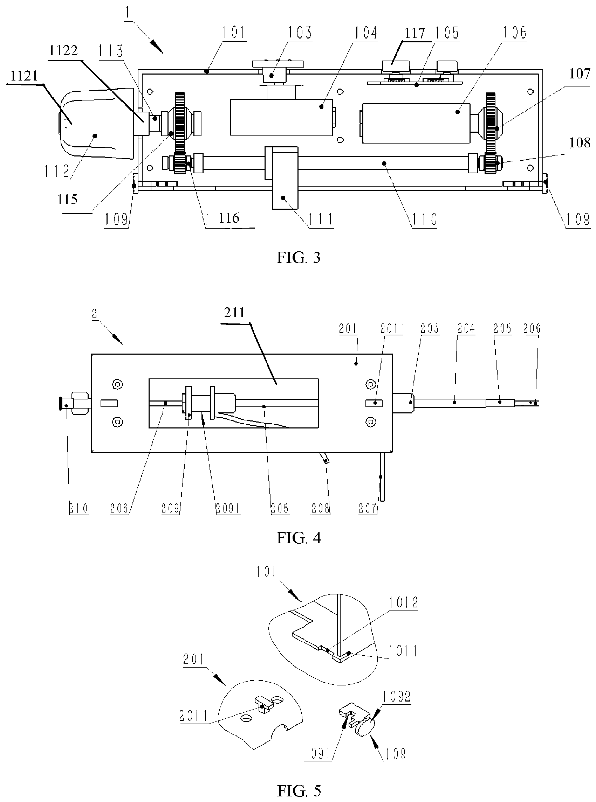

Referring to FIGS. 2 and 3, the handle 1 includes a handle housing 10, a driving device, and a transmission component. The handle housing 10 includes the first housing 101 and the second housing 102. In other embodiments, the handle housing 10 can also be adopted as an integrated housing, which is not particularly limited in the present application. A bottom of the handle housing 10 is provided with a hollow first slot 114. A first connector 109 is provided on the handle housing 10. The handle housing 10 is detachably connected to the catheter assembly 2 through the first connector 109.

The driving device is arranged inside and outside the handle housing 10. The handle 1 can be a pure electric handle or a pure manual handle, or an electric and manual hybrid handle. In an embodiment, the driving device includes both an electric driving device and a manual driving device. The electric driving device includes a switch 103, a power supply 104, a control board 105, and a motor 106. The manual driving device includes a manual rotating member 112 and a gear shaft 113.

The transmission component includes a first large gear 107, a first pinion gear 108, a second large gear 115, a second pinion gear 116, a screw rod 110, and a driving member 111.

Specifically, the switch 103 is electrically connected to the power supply 104. The power supply 104 is electrically connected to the control board 105. The control board 105 is electrically connected to the motor 106. The power supply 104 and the motor 106 are located inside the handle housing 10, and the power supply 104 provides a power source for the handle 1. In order to facilitate operation, one part of the switch 103 is embedded in the handle housing 10, and the other part thereof is exposed to the handle housing 10. The exposed part of the switch 103 is provided with an operation key. The operation key is used to control the on/off of the circuit between the power supply 104 and the motor 106. Similar to the switch 103, one part of the control board 105 is embedded in the handle housing 10, and the other part thereof is exposed to the handle housing 10. The exposed part of the control board 105 is provided with a control key 117. The control key 117 is used to control the operating state of the motor 106, including starting, advancing, reversing, stopping, etc. An end of the motor 106 has an output end. The first large gear 107 is coaxially arranged with and circumferentially fixedly connected to the output end of the motor 106. The first pinion gear 108 is meshed with the first large gear 107. The first pinion gear 108 is coaxial with the screw rod 110, and the first pinion gear 108 is fixedly connected to a distal end of the screw rod 110. A fixing component, such as a nut, is matched and connected to the screw rod 110. The driving member 111 is fixedly connected to the nut of the screw rod 110. In such a configuration, the motor 106 is arranged in parallel with the screw rod 110, such that a length of the handle 1 can be shortened, thereby reducing a size of the handle. The manual rotating member 112 includes a manual control member 1121 and a rotating shaft 1122 that are fixedly connected. For the convenience of manual operation, the manual rotating member 112 is arranged at a proximal end of the handle housing 10. The manual control member 1121 is arranged at an outer side of the proximal end of the handle housing 10. The rotating shaft 1122 passes through the handle housing 10, and then is coaxially arranged with and fixedly connected to the gear shaft 113. The gear shaft 113 and the second large gear 115 are arranged coaxially, and fixedly connected circumferentially. The second large gear 115 is meshed with the second pinion gear 116. The second pinion gear 116 is coaxially arranged with the screw rod 110, and the second pinion gear 116 is fixedly connected to a proximal end of the screw rod 110.

In an electric mode, a rotational movement of the output end of the motor 106 drives the first large gear 107 to rotate, and then drives the first pinion gear 108 to rotate through the gear meshing, which in turn drives the screw rod 110 to rotate, so that the driving member 111 fixed on the nut is moved linearly. In a manual mode, a rotational movement of the manual rotating member 112 drives the second large gear 115 to rotate, and then drives the second pinion gear 116 to rotate through the gear meshing, which in turn drives the screw rod 110 to rotate, so that the driving member 111 fixed on the nut is moved linearly. The hybrid setting of electric mode and manual mode can play a double insurance role. If one mode fails, another mode will replace it, and the handle can continue to work, reducing the probability of handle failure.

In one of other embodiments, the output end of the motor 106 is coaxially arranged with the screw rod 110, and the screw rod 110 is directly connected to the output end of the motor 106 through a coupling (not shown), without the transmission of the first large gear 107 and the first pinion gear 108. Specifically, one end of the coupling is fixedly connected to the output end of the motor 106, another end of the coupling is fixedly connected to the distal end of the screw rod 110, and the remaining structures are the same as those described in above embodiments. In such a configuration, the rotational movement of the output end of the motor 106 directly drives the screw rod 110 to rotate, so that the driving member 111 fixed on the nut is driven to move linearly. In this embodiment, the output end of the motor 106 is coaxially arranged with the screw rod 110, and there is no need for transmission through the meshing of large and pinion gears, which can simplify the parts.

In one of other embodiments, a driving mode of the handle may be a pure electric mode or a pure manual mode. The following components can be removed from the pure electric handle: the manual rotating member 112, the gear shaft 113, the second large gear 115, and the second pinion gear 116. The following components can be removed from the pure manual handle: the switch 103, the power supply 104, the control board 105, the motor 106, the first large gear 107, and the first pinion gear 108.

In one of other embodiments, the motor is a linear motor with a motor shaft (not shown). The driving member is fixed on the motor shaft. The motor shaft reciprocates linearly in an axial direction under the control of the control board, thereby driving the driving member to move linearly. In this embodiment, there is no need for gears for meshing transmission, and no need to provide a screw rod, which simplifies the internal structure of the handle. Therefore, the handle according to the present application is applicable for various driving structures, and can be configured according to actual conditions, as long as it is ensured that the driving member 111 can linearly reciprocate in the axial direction.

Referring to FIG. 4, the catheter assembly 2 is located outside the handle 1, and includes a catheter assembly housing 20, an outer tube 205, an inner tube assembly 206, an outer tube evacuation tube 208, an outer tube base 209, and an inner tube evacuation tube 210. Further, the catheter assembly 2 further includes a stabilizing tube base 203 and a stabilizing tube 204. In order to facilitate assembly, the catheter assembly housing 20 includes the third housing 201 and the fourth housing 202. A second connector is provided on the catheter assembly housing 20. The second connector is detachably connected to the first connector 109, so that the handle 1 and the catheter assembly 2 can be quickly assembled with and disassembled from each other. A top surface of the catheter assembly housing 20 is provided with a hollow second slot 211. A position and a shape of the second slot 211 correspond to those of the first slot 114. The first slot 114 and the second slot 211 are correspondingly provided on the handle housing 10 and the catheter assembly housing 20, which, on the one hand, realizes the detachable connection between the driving member 111 and the outer tube base 209 at the first slot 114 and the second slot 211, and on the other hand, provides the movement space required for driving the outer tube base 209 to move linearly when the driving member 111 moves linearly.

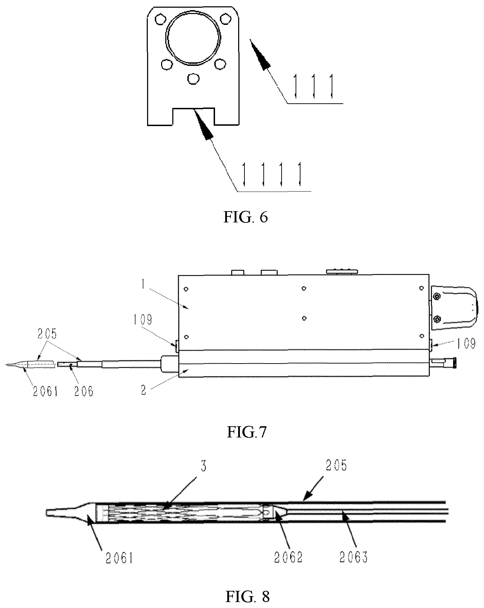

Referring to FIG. 8, the inner tube assembly 206 includes an inner tube 2063, a fixed head 2062, and a conical head 2061 that are connected in sequence from a proximal end to a distal end. Further, a proximal end of the inner tube 2063 is further provided with an inner tube evacuation tube 210. A proximal end of the inner tube evacuation tube 210 is arranged outside the catheter assembly housing 20. The proximal end of the inner tube 2063 is connected to a distal end of the inner tube evacuation tube 210, and then is fixed to a proximal end of the catheter assembly housing 20 by a fixing member. Air in the inner tube 2063 can be evacuated by injecting liquid into the inner tube evacuation tube 210 during the operation. The inner tube 2063 passes through the outer tube base 209 and is located in the outer tube. A distal end of the outer tube base 209 is fixedly connected to the outer tube 205, and the outer tube base 209 and the outer tube 205 remain stationary with respect to each other in the axial direction. When the outer tube base 209 moves in the axial direction, the outer tube 205 is driven to move accordingly. The outer tube base 209 is further provided with the outer tube evacuation tube 208 that communicates with the outer tube 205. The outer tube evacuation tube 208 extends out of and is exposed to the catheter assembly housing 20. During the operation, the air between the outer tube 205 and the inner tube 2063 can be evacuated when liquid is injected into the outer tube evacuation tube 208. The outer tube base 209, the outer tube 205, and the outer tube evacuation tube 208 are fixedly connected and can move linearly in the axial direction.

In a preferred embodiment, the outer tube 205 is further sleeved with the stabilizing tube base 203 and the stabilizing tube 204, and the stabilizing tube base 203 is fixed on a distal end of the catheter assembly housing 20. Preferably, a distal end of the stabilizing tube base 203 is in sealed connection with a proximal end of the stabilizing tube 204. A distal end of the outer tube 205 passes through the stabilizing tube base 203 and the stabilizing tube 204. The stabilizing tube 204 is sleeved outside the outer tube 205, and covers a part of the outer tube 205. The outer tube 205 can move linearly in the axial direction along an outer side of the inner tube assembly 206 and inner sides of the stabilizing tube 204 and the stabilizing tube base 203. Further, the stabilizing tube base 203 is further in sealed connection with a stabilizing tube evacuation tube 207 communicating with the stabilizing tube 204. The stabilizing tube evacuation tube 207 extends out of and is exposed to the catheter assembly housing 20. Air between the outer tube 205 and the stabilizing tube 204 can be evacuated by injecting liquid into the stabilizing tube evacuation tube 207 during the operation. The stabilizing tube 204 supports the outer tube 205 through the stabilizing tube base 203, provides an acting point for the longer catheter delivery system, and provides an acting point for the movement of the outer tube 205 in the complex blood vessels of the human body during the operation, preventing the outer tube 205 from rubbing against the blood vessels. In other embodiments, when the outer tube 205 has sufficient strength, components such as the stabilizing tube 204, the stabilizing tube evacuation tube 207, and the stabilizing tube base 203 may be omitted, and the structure of the inner tube assembly will be further simplified.

Referring to FIGS. 2, 4, and 6, the driving member 111 is fixed to the outer tube base 209, and is detachably connected to the outer tube base 209. In an embodiment, a bottom of the driving member 111 is provided with a first groove 1111, and the outer tube base 209 is provided with a second groove 2091. The first groove 1111 and the second groove 2091 cooperate with each other, so that the driving member 111 can be sleeved on the outer tube base 209 for insertion. Specifically, the second groove 2091 is grooved along the second direction, and the second groove 2091 has limiting components on both sides in the axial direction. The movement of the driving member 111 with respect to the outer tube base 209 in the axial direction is limited by the limiting components on both sides in the axial direction, so that the driving member 111 and the outer tube base 209 can remain stationary with respect to each other in the axial direction. Accordingly, the first groove 1111 is grooved along the axial direction, and the first groove 1111 has limiting components on both sides in the second direction. The relative movement of the driving member 111 in the second direction is limited by the limiting components on both sides, so that the driving member 111 and the outer tube base 209 are stably and fixedly connected on the horizontal plane, and in turn, the linear reciprocating movement of the driving member 111 drives the outer tube base 209 to reciprocate linearly, and finally the outer tube 205 fixedly connected to the outer tube base 209 reciprocates linearly. Meanwhile, in the third direction, the driving member 111 is detachable from the outer tube base 209.

Referring to FIGS. 2, 3, and 5, the detachable connection of the first connector 109 and the second connector on the catheter assembly housing 20 can be a slot connection, a threaded connection, a snap connection, a pin connection, or a spline connection etc. In an embodiment, taking the slot connection as an example, the first connector 109 has a detaching button 1092 and a third slot 1091, and the second connector is a protrusion 2011 having a limiting component in the third direction. The protrusion 2011 may be T-shaped, 7-shaped or inverted L-shaped. The protrusion 2011 shown in FIG. 5 is T-shaped. The handle housing 10 is provided with a first through hole 1011 and a second through hole 1012. When the handle 1 is connected to the catheter assembly 2, a proximal end of the first connector 109 passes through the first through hole 1011 so that the third slot 1091 is located at the inner side of the handle housing 10, and the detaching button 1092 is located at the outer side of the handle housing 10. The handle housing 10 has a locking position and a detaching position (not shown) in the second direction. The detaching button 1092 can be toggled between the locking position and the detaching position. When the detaching button 1092 is in the detaching position, the protrusion 2011 can pass through the second through hole 1012 and then be located at the inner side of the handle housing 10, and when the protrusion 2011 toggles the detaching button 1092 in the second direction to the locking position, the third slot 1091 is engaged with the protrusion 2011, so that the catheter assembly housing 20 cannot be separated from the handle housing 10.

At the same time, a position of the driving member 111 on the screw rod 110 is adjust so that the first groove 1111 (as shown in FIG. 6) can be engaged with the second groove 2091 on the outer tube base 209 (as shown in FIG. 4), so as to realize the synchronization of the linear movements of the handle 1 and the catheter assembly 2, and the connection between the housing of the handle 1 and the housing of the catheter assembly 2. When the handle 1 is detached from the catheter assembly 2, the detaching button 1092 is toggled towards the other direction to the detaching position, so that the third slot 1091 is disengaged with the protrusion 2011 in the second direction, and the catheter assembly housing 20 can be separated from the handle housing 10 in the third direction, while the first groove 1111 and the second groove 2091 are disengaged in the third direction.

In this embodiment, the detaching button 1092 is toggled in the second direction to realize the slot connection. In other embodiments, the slot connection can also be realized by pushing and pulling the first connector 109 in the first direction, namely, the axial direction.

Therefore, the handle 1 and the catheter assembly 2 in the delivery system according to the present application realize the detachable connection between the handle housing 10 and the catheter assembly housing 20 through the two first connectors 109 and the second connectors provided on both sides thereof. In addition, the driving member 111 and the outer tube base 209 are also detachably connected, but it is necessary to ensure that after the driving member 111 is connected to the outer tube base 209, there is no relative displacement in the direction in which the driving member 111 is moved linearly, so as to convert the linear movement of the driving member 111 to the linear movements of the outer tube base 209 and the outer tube 205.

Referring to FIGS. 7 and 8, a distal end of the catheter assembly 2 includes the outer tube 205 and the inner tube assembly 206 that are sleeved in sequence. The outer tube 205 is sleeved outside the inner tube assembly 206. The inner tube assembly 206 includes the inner tube 2063, the fixed head 2062, and the conical head 2061 which are connected in sequence from the proximal end to the distal end. The inner tube 2063 on the inner tube assembly 206 is fixedly connected to the proximal end of the catheter assembly housing 20 on the catheter assembly 2. Six degrees of freedom of the fixed head 2062 are limited to fix and support an implant 3. By driving the outer tube 205 to linearly reciprocate in the axial direction, the outer tube 205 linearly reciprocates in the axial direction with respect to the inner tube assembly 206, so as to realize operations such as loading, releasing and recycling the implant 3.

If it is intended to replace the failed handle, the two first connectors 109 are toggled to the detaching position, the failed handle 1 can be directly separated from the catheter assembly 2, and then, a new handle 1 is inserted into the catheter assembly 2, and then the driving member 111 is aligned with the outer tube base 209, and then the two first connectors 109 can be toggled to the locking position again.

If it is intended to reuse the handle 1 to reduce the cost of each operation, after the operation, the handle 1 is separated from the catheter assembly 2, and the catheter assembly 2 is disposed of as waste. After the handle 1 is sterilized, the handle 1 can be used with a new catheter assembly 2 again.

To sum up, the handle and delivery system for implants according to this embodiment at least have the following advantages:
1) The handle 1 and the catheter assembly 2 can be quickly assembled and disassembled, which can quickly deal with the safety risk of handle failure and avoid more trauma to the patient caused by the change from the interventional surgery to the surgical operation due to the handle problem. In addition, the handle can be reused, the cost of each operation can be reduced, and the economic burden of the patient can be reduced.
2) The motor 106 is arranged in parallel with the screw rod 110, which can shorten the length of the handle 1 and reduce the size of the handle.
3) The structural arrangement of the catheter assembly 2 is centralized. That is, the components in contact with blood, such as the outer tube assembly, the inner tube assembly, the evacuation tubes, are all centrally arranged in the catheter assembly 2. The handle 1 and the catheter assembly 2 are individually assembled into an integral assembly, which are independent of each other, and thus the manual and electric operations of the handle 1 and the evacuating operation of the catheter assembly 2 do not affect each other. The manually-operated rotating members, the electrically-operated buttons and the evacuation tubes of the catheter assembly are far apart in space, and have no moving contact and will not interfere with each other's operations. Moreover, the evacuation tubes are located in the catheter assembly 2 instead of the handle 1, so there is no need to consider the sealing and leakage problems caused by detaching the handle 1, the structure is simple and reliable, and the handle 1 can be safely reused.
4) The distal end of the catheter assembly 2 is not obstructed by other parts (such as rotating members) in the handle, leaving more operating space for functional expansion such as catheter closure after the implant is released and rapid retraction of the inner tube.
5) Whether the handle 1 is electric, manual or hybrid of electric and manual, the handle 1 can be manufactured to be detachable from the catheter assembly 2, and has a wide range of adaptability.

### Second Embodiment

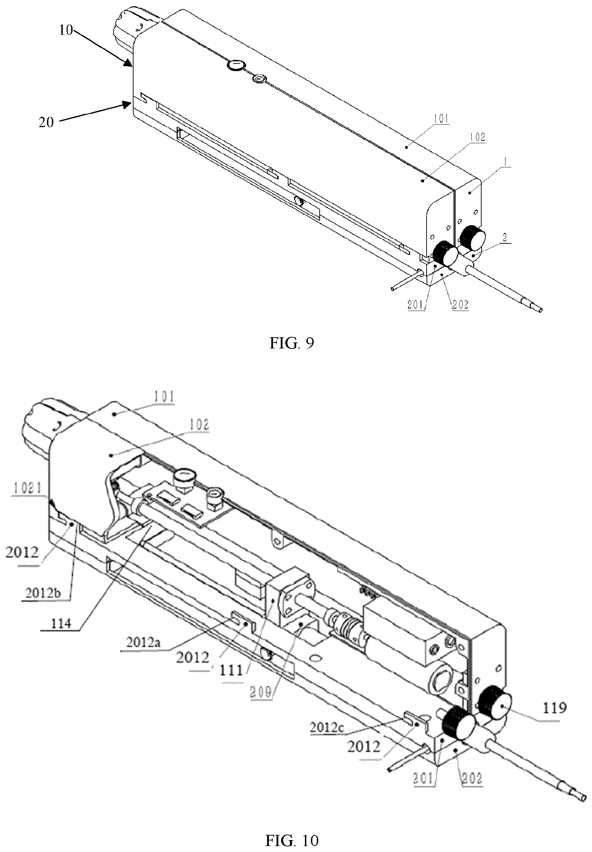

Referring to FIG. 9, a delivery system for delivering implants according to this embodiment includes a handle 1 and a catheter assembly 2. The catheter assembly 2 is located outside the handle 1. The catheter assembly 2 and the handle 1 are detachably connected. In order to facilitate assembly, preferably, the handle 1 includes a first housing 101 and a second housing 102. The first housing 101 and the second housing 102 are fixedly connected. The catheter assembly 2 includes a third housing 201 and a fourth housing 202. The third housing 201 and the fourth housing 202 are fixedly connected.

Referring to FIGS. 10, 11, and 13, the handle 1 includes a handle housing 10, a driving device, and a transmission component. The handle housing 10 includes the first housing 101 and the second housing 102. In other embodiments, the handle housing 10 can also adopted as an integrated housing, which is not particularly limited in the present application. A bottom of the handle housing 10 is provided with a hollow first slot 114. A first engaging component 118 is provided on the handle housing 10. The handle housing 10 is detachably connected to the catheter assembly 2 through the first engaging component 118 and a fastening component 119.

The driving device is arranged inside and outside the handle housing 10. The handle 1 can be a pure electric handle or a pure manual handle, or an electric and manual hybrid handle. In an embodiment, the driving device includes both an electric driving device and a manual driving device. The electric driving device includes a switch 103, a power supply 104, a control board 105, and a motor 106. The manual driving device includes a manual rotating member 112 and a gear shaft 113.

The transmission component includes a coupling 120 and a screw rod 110.

Specifically, the switch 103 is electrically connected to the power supply 104. The power supply 104 is electrically connected to the control board 105. The control board 105 is electrically connected to the motor 106. The power supply 104 and the motor 106 are located inside the handle housing 10, and the power supply 104 provides a power source for the handle 1. In order to facilitate operation, one part of the switch 103 is embedded in the handle housing 10, and the other part thereof is exposed to the handle housing 10. The exposed part of the switch 103 is provided with an operation key. The operation key is used to control the on/off of the circuit between the power supply 104 and the motor 106. Similar to the switch 103, one part of the control board 105 is embedded in the handle housing 10, and the other part thereof is exposed to the handle housing 10. The exposed part of the control board 105 is provided with a control key 117. The control key 117 is used to control the operating state of the motor 106, including starting, advancing, reversing, stopping, etc. An end of the motor 106 has an output end 1061. The output end 1061 of the motor 106 is coaxially arranged with the screw rod 110. The screw rod 110 is connected to the output end 1061 of the motor 106 directly through the coupling 120. Specifically, one end of the coupling 120 is fixedly connected to the output end 1061 of the motor 106, and another end of the coupling 120 is fixedly connected to a distal end of the screw rod 110. In such a configuration, the rotational movement of the output end 1061 of the motor 106 directly drives the screw rod 110 to rotate, so that the driving member 111 fixed on the nut is driven to move linearly in the first slot 114. The manual rotating member 112 includes a manual control member 1121 and a rotating shaft 1122 that are fixedly connected. For the convenience of manual operation, the manual rotating member 112 is arranged at a proximal end of the handle housing 10, and the manual control member 1121 is arranged at an outer side of the proximal end of the handle housing 10. The rotating shaft 1122 passes through the handle housing 10, and is coaxially arranged with and circumferentially fixedly connected with the gear shaft 113. The gear shaft 113 and a large gear 122 are arranged coaxially and fixedly connected circumferentially. The large gear 122 is meshed with a pinion gear 121. The pinion gear 121 is coaxially arranged with the screw rod 110, and is fixedly connected to a proximal end of the screw rod 110. In this embodiment, the output end 1061 of the motor 106 and the screw rod 110 are coaxially arranged, and there is no need for transmission through the meshing of large and pinion gears, which can simplify the parts.

In an electric mode, a rotational movement of the output end 1061 of the motor 106 drives the screw rod 110 to rotate, so that the driving member 111 fixed on the nut is moved linearly. In a manual mode, a rotational movement of the manual rotating member 112 drives the large gear 122 to rotate, and then drives the pinion gear 121 to rotate through the gear meshing, which in turn drives the screw rod 110 to rotate, so that the driving member 111 fixed on the nut is moved linearly. The hybrid setting of electric mode and manual mode can play a double insurance role. If one mode fails, another mode will replace it, and the handle can continue to work, reducing the probability of handle failure.

In one of other embodiments, a driving mode of the handle may be a pure electric mode or a pure manual mode. The following components can be removed from the pure electric handle: the manual rotating member 112, the gear shaft 113, the large gear 122, and the pinion gear 121. The following components can be removed from the pure manual handle: the switch 103, the power supply 104, the control board 105, the motor 106, and the coupling 120.

In one of other embodiments, the motor is a linear motor with a motor shaft (not shown). The driving member is fixed on the motor shaft. The motor shaft reciprocates linearly along an axial direction under the control of the control board 105, thereby driving the driving member to move linearly. In this embodiment, there is no need to provide a screw rod, which simplifies the internal structure of the handle. Therefore, the handle according to the present application is applicable for various driving structures, and can be configured according to actual conditions, as long as it is ensured that the driving member 111 can linearly reciprocate in the axial direction.

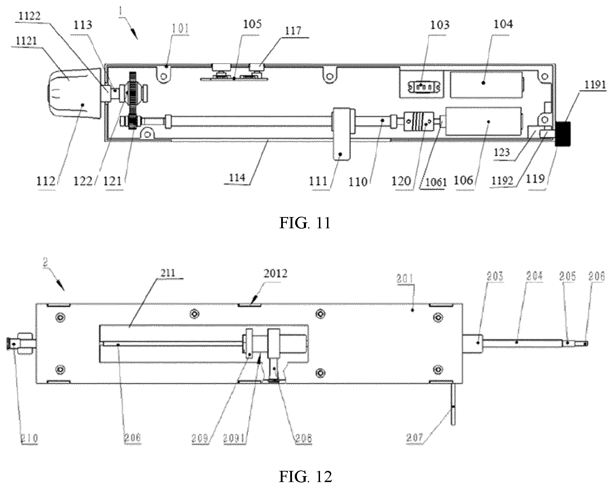

Referring to FIGS. 9 and 12, the catheter assembly 2 is located outside of the handle 1, and includes a catheter assembly housing 20, an outer tube 205, an inner tube assembly 206, an outer tube evacuation tube 208, an outer tube base 209, and an inner tube evacuation tube 210. Further, the catheter assembly 2 further includes a stabilizing tube base 203 and a stabilizing tube 204. In order to facilitate assembly, the catheter assembly housing 20 includes the third housing 201 and the fourth housing 202. A second engaging component 2012 is provided on the catheter assembly housing 20. The second engaging component 2012 is detachably connected to the first engaging component 118 in the first direction, so that the handle 1 and the catheter assembly 2 can be quickly assembled with and disassembled from each other. A top surface of the catheter assembly housing 20 is provided with a hollow second slot 211. A position and a shape of the second slot 211 correspond to those of the first slot 114. The first slot 114 and the second slot 211 correspondingly provided on the handle housing 10 and the catheter assembly housing 20, not only realize the detachable connection between the driving member 111 and the outer tube base 209 at the first slot 114 and the second slot 211, but also provide the movement space required for driving the outer tube base 209 to move linearly, that is, driving the outer tube base 209 to move towards the proximal end or the distal end of the catheter assembly 2 in the second slot 211, when the driving member 111 moves linearly.

Referring to FIGS. 10, 12, and 14, the driving member 111 is fixed to and detachably connected to the outer tube base 209. In an embodiment, a bottom of the driving member 111 is provided with a first groove 1111, and the outer tube base 209 is provided with a second groove 2091. The first groove 1111 and the second groove 2091 cooperate with each other, so that the driving member 111 can be sleeved on the outer tube base 209 for insertion. Specifically, the second groove 2091 is grooved along the second direction, and the second groove 2091 has limiting components on both sides in the axial direction. The movement of the driving member 111 with respect to the outer tube base 209 in the axial direction is limited by the limiting components on both sides in the axial direction, so that the driving member 111 and the outer tube base 209 can remain stationary with respect to each other in the axial direction. Accordingly, the first groove 1111 is grooved along the axial direction, and the first groove 1111 has limiting components on both sides in the second direction. The relative movement of the driving member 111 in the second direction is limited by the limiting components on both sides, so that the driving member 111 and the outer tube base 209 are stably and fixedly connected on the horizontal plane, and in turn, the linear reciprocating movement of the driving member 111 drives the outer tube base 209 to reciprocate linearly, and finally the outer tube 205 fixedly connected to the outer tube base 209 reciprocates linearly. Meanwhile, in the third direction, the driving member 111 is detachable from the outer tube base 209. The connection means between the driving member 111 and the outer tube base 209 are not particularly limited in the present application, as long as the detachable connection between the driving member 111 and the catheter assembly 2 in the third direction can be achieved, such as other concave-convex shape-fit connection, or pin connection.

Referring to FIGS. 12, 15, and 8, the inner tube assembly 206 includes an inner tube 2063, a fixed head 2062, and a conical head 2061 that are connected in sequence from a proximal end to a distal end. Further, a proximal end of the inner tube 2063 is further provided with an inner tube evacuation tube 210. A proximal end of the inner tube evacuation tube 210 is arranged outside the catheter assembly housing 20. The proximal end of the inner tube 2063 is connected to a distal end of the inner tube evacuation tube 210, and then is fixed to a proximal end of the catheter assembly housing 20 by a fixing component. Air in the inner tube 2063 can be evacuated by injecting liquid into the inner tube evacuation tube 210 during the operation. The inner tube 2063 passes through the outer tube base 209 and is located in the outer tube. A distal end of the outer tube base 209 is fixedly connected to the outer tube 205, and the outer tube base 209 and the outer tube 205 remain stationary with respect to each other in the axial direction. When the outer tube base 209 moves in the axial direction, the outer tube 205 is driven to move accordingly. The outer tube base 209 is further provided with the outer tube evacuation tube 208 that communicates with the outer tube 205. The outer tube evacuation tube 208 extends out of and is exposed to the catheter assembly housing 20. During the operation, the air between the outer tube 205 and the inner tube 2063 can be evacuated when liquid is injected into the outer tube evacuation tube 208. The outer tube base 209, the outer tube 205, and the outer tube evacuation tube 208 are fixedly connected and can move linearly in the axial direction.

In a preferred embodiment, the outer tube 205 is further sleeved with the stabilizing tube base 203 and the stabilizing tube 204, and the stabilizing tube base 203 is fixed on a distal end of the catheter assembly housing 20. Preferably, a distal end of the stabilizing tube base 203 is in sealed connection with a proximal end of the stabilizing tube 204. A distal end of the outer tube 205 passes through the stabilizing tube base 203 and the stabilizing tube 204. The stabilizing tube 204 is sleeved outside the outer tube 205, and covers a part of the outer tube 205. The outer tube 205 can move linearly in the axial direction along an outer side of the inner tube assembly 206 and inner sides of the stabilizing tube 204 and the stabilizing tube base 203. Further, the stabilizing tube base 203 is further in sealed connection with a stabilizing tube evacuation tube 207 communicating with the stabilizing tube 204. The stabilizing tube evacuation tube 207 extends out of and is exposed to the catheter assembly housing 20. Air between the outer tube 205 and the stabilizing tube 204 can be evacuated by injecting liquid into the stabilizing tube evacuation tube 207 during the operation. The stabilizing tube 204 supports the outer tube 205 through the stabilizing tube base 203, provides an acting point for the longer catheter delivery system, and provides an acting point for the movement of the outer tube 205 in the complex blood vessels of the human body during the operation, preventing the outer tube 205 from rubbing against the blood vessels. In other embodiments, when the outer tube 205 has sufficient strength, components such as the stabilizing tube 204, the stabilizing tube evacuation tube 207, and the stabilizing tube base 203 may be omitted, and the structure of the inner tube assembly will be further simplified.

Referring to FIGS. 9, 10, and 13, the detachable connection between the first engaging component 118 on the handle housing 10 and the second engaging component 2012 on the catheter assembly housing 20 is a slot connection. The first engaging component 118 is provided on an edge of the bottom of the handle housing 10, and the second engaging component 2012 is provided on an edge of the top of the catheter assembly housing 20. At least two, preferably four or six first engaging components 118 and second engaging components 2012 may be provided, which are specifically determined according to lengths of the handle 1 and the catheter assembly 2 in the first direction. If the lengths of the handle housing 10 and the catheter assembly housing 20 in the first direction are smaller, four first engaging components 118 and four second engaging components 2012 may preferably be provided. If the lengths of the handle housing 10 and the catheter assembly housing 20 in the first direction are larger, six first engaging components 118 and six second engaging components 2012 may preferably be provided. Further, a plurality of first engaging components 118 are evenly distributed on the edge of the bottom of the handle housing 10 along the first direction, and a plurality of second engaging components 2012 are evenly distributed on the edge of the top of the catheter assembly housing 20 along the first direction. In an embodiment, the second engaging component 2012 includes a first protruding portion 2012a and a second protruding portion 2012b that are connected to each other. The first protruding portion 2012a extends in the first direction, and the second protruding portion 2012b extends in the third direction. A first opening 2012c is formed between the first protruding portion 2012a, the second protruding portion 2012b and the edge of the catheter assembly housing 20. The first opening 2012c faces the proximal end of the catheter assembly 2, and the advantage of such configuration is that the handle 1 can be detached from the catheter assembly 2 even when the distal end of the outer tube and the distal end (conical head) of the inner tube are closed. If the first opening 2012c is arranged towards the distal end, in this case, the inner tube assembly 206 cannot be retracted and cannot be disassembled. The first engaging component 118 includes a first engaging strip 1021 or a third engaging strip 1022 and a first engaging slot 1023. The first engaging strip 1021 or the third engaging strip 1022 protrudes in the second direction and is arranged in the first engaging slot 1023. The first engaging strip 1021 is arranged at the proximal end of the handle housing 10. Preferably, the first engaging slot 1023 extends in the first direction to form a first guiding rail 1024. The second engaging component 2012 can slide along the first guiding rail 1024 in the first direction, so that the first opening 2012c can be easily snapped on the engaging strip 1021. Further, a plurality of first engaging slots 1023 extend to form a continuous first guiding rail 1024, which is more convenient for processing. Preferably, the first protruding portion 2012a and the second protruding portion 2012b on the edge of the top of the catheter assembly housing 20 are engaged in the first engaging slot 1023 of the handle housing 10, and the first opening 2012c of the second engaging components 2012 is snapped on the first engaging strip 1021 or the third engaging strip 1022, which allows the catheter assembly 2 to slide with respect to the handle 1 along the first guiding rail 1024 in the first direction, while limiting the movement of the catheter assembly 2 in the third direction. The length of the first opening 2012c in the axial direction should be smaller than the relative displacement length of the inner tube and the outer tube, so as to satisfy the small relative movement between the inner tube and the outer tube, and further protect the delivery system or a stent crimped therein.

Continuing to refer to FIGS. 10 and 11, in order to fix the handle 1 and the catheter assembly 2 in the first direction, in an embodiment, a fastening component 119 is provided on a side of the distal end of the handle housing 10. The fastening component 119 is preferably a threaded knob, including a threaded portion 1192 and a knob portion 1191. The knob portion 1191 is provided at the outer side of the handle housing 10, and the threaded portion 1192 passes through the handle housing 10 and is threadedly connected to a fixing member 123 provided in the handle housing 10. A surface of the proximal end of the knob portion 1191 is in close contact with a surface of the distal end of the catheter assembly housing 20, to horizontally fix the catheter assembly 2 with respect to the handle 1. In other embodiments, the threaded connection of the threaded knob can also be replaced with a pin connection or a snap connection, so as to realize the relative fixation of the handle housing 10 and the catheter assembly housing 20 in the first direction.

In this embodiment, through the slot connection between the first engaging component 118 and the second engaging component 2012, the handle 1 and the catheter assembly 2 limit the relative sliding in the first direction and the fixing in the third direction of the handle housing 10 and the catheter assembly housing 20, which in turn realizes the detachable connection between the handle 1 and the catheter assembly 2 in the first direction. The protruding shape of the second engaging component 2012, the slot shape of the second engaging component 2012, the shape of the guiding rail, the setting means and the number of the foregoing three are not limited in the present application, and which can be set by those skilled in the art according to the needs, as long as the detachable connection between the handle 1 and the catheter assembly 2 in the first direction can be realized.

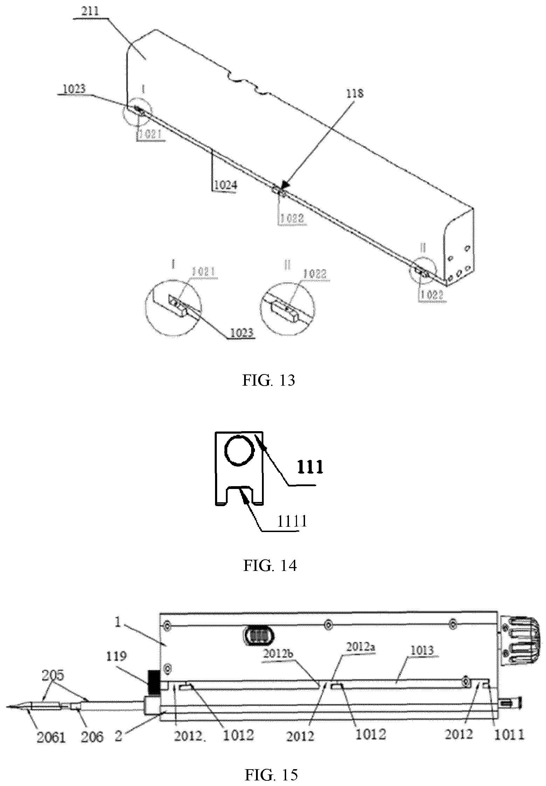

Referring to FIGS. 12 and 14, when the handle 1 is connected to the catheter assembly 2, the axial position of the outer tube base 209 on the inner tube assembly 206 is firstly adjusted, so that the second groove 2091 on the outer tube base 209 is slightly close to the proximal end of the handle 1 with respect to the first groove 1111 on the driving member 111. Then, the first groove 1111 is engaged with the second groove 2091, and an upper surface of the catheter assembly housing 20 is abutted against a lower surface of the handle housing 10, to ensure that after being connected to the driving member 111, the outer tube base 209 does not have relative displacement in the direction in which the driving member 111 is linearly moved, so that the linear movement of the driving member 111 is converted into the linear movements of the outer tube base 209 and the outer tube 205. Then, the catheter assembly 2 slides towards the proximal end of the handle 1 as far as possible, and the first opening 2012c of the second engaging component 2012 is snapped on the first engaging strip 1021 or the third engaging strip 1022. In this case, the outer tube base 209 and the outer tube 205 are moved towards the distal end of the catheter assembly 2 with respect to the inner tube 2063. Finally, the fastening component 119 (threaded knob) on the handle 1 is tightened, so that the surface of the proximal end of the fastening component 119 is abutted against the surface of the distal end of the catheter assembly housing 20 to realize that the catheter assembly 2 is fixed in the first direction with respect to the handle 1.

When the handle 1 is detached from the catheter assembly 2, the fastening component 119 is untightened to the detaching position, and the catheter assembly 2 slides towards the distal end relative to the handle 1. In this case, the outer tube base 209 and the outer tube 205 are moved towards the proximal end of the catheter assembly 2 with respect to the inner tube 2063, and the first engaging component 118 is separated from the second engaging component 2012. Then, the catheter assembly housing 20 can be separated from the handle housing 10 in the third direction, while the first groove 1111 and the second groove 2091 are disengaged in the third direction.

In another embodiment, the structure of the first engaging component 118 can also be interchanged with the structure of the second engaging component 2012 by those skilled in the art. That is, the first engaging component 118 includes a third protruding portion and a fourth protruding portion that are connected to each other. The third protruding portion extends in the first direction, and the fourth protruding portion extends in the third direction. A second opening is formed between the third protruding portion, the fourth protruding portion and the edge of the bottom of the handle housing. Preferably, the second opening faces the distal end of the handle 1. The second engaging component 2012 includes a second engaging slot and a second engaging strip. The second engaging strip protrudes in the second direction and is arranged in the second engaging slot. Further, the second engaging slot extends in the first direction to form a second guiding rail. Correspondingly, a fastening component, such as a threaded knob, is provided on a side of the proximal end of the catheter assembly housing 20. When being tightened, the surface of the fastening component is abutted against the surface of the proximal end of the handle housing 10, to realize that catheter assembly housing 20 is fixed with respect to the handle housing 10 in the first direction.

Therefore, the handle 1 and the catheter assembly 2 in the delivery system according to the present application realizes the detachable connection between the handle housing 10 and the catheter assembly housing 20, through the plurality of first engaging components 118 and second engaging components 2012 arranged on the edge of the housing, as well as the fastening component 119 arranged on the side of the handle 1 or the catheter assembly 2. In addition, the driving member 111 and the outer tube base 209 are also detachably connected, but it is necessary to ensure that after the driving member 111 is connected to the outer tube base 209, there is no relative displacement in the direction in which the driving member 111 is moved linearly, so that the linear movement of the driving member 111 is converted into the linear movements of the outer tube base 209 and the outer tube 205.

Referring to FIGS. 15 and 8, the distal end of the catheter assembly 2 includes the outer tube 205 and the inner tube assembly 206 that are sleeved in sequence. The outer tube 205 is sleeved outside the inner tube assembly 206. The inner tube assembly 206 includes the inner tube 2063, the fixed head 2062, and the conical head 2061 that are connected in sequence from the proximal end to the distal end. The inner tube 2063 on the inner tube assembly 206 is fixedly connected to the proximal end of the catheter assembly housing 20 on the catheter assembly 2. Six degrees of freedom of the fixed head 2062 are limited to fix and support an implant 3. By driving the outer tube 205 to linearly reciprocate in the axial direction, the outer tube 205 linearly reciprocates in the axial direction with respect to the inner tube assembly 206, so as to realize operations such as loading, releasing and recycling the implant 3.

If it is intended to replace the failed handle, the fastening component 119 is untightened to the detaching position, the failed handle 1 can be directly separated from the catheter assembly 2 by sliding towards the proximal end relative to the catheter assembly 2. In this case, the distal end of the outer tube 205 is normally separated from the conical head 2061, and then, the driving member 111 of a new handle 1 is aligned with the outer tube base 209, and then, the handle 1 slides towards the distal end relative to the catheter assembly 2 and cooperates with the catheter assembly 2, and the fastening component 119 is tightened to realize that the handle 1 and the catheter assembly 2 are fixed in the first direction.

If it is intended to reuse the handle 1 to reduce the cost of each operation, after the operation, the handle 1 is separated from the catheter assembly 2, and the catheter assembly 2 is disposed of as waste. After the handle 1 is sterilized, the handle 1 can be used with a new catheter assembly 2 again.

To sum up, the handle and delivery system for implants according to this embodiment at least have the following advantages:
1) The handle 1 and the catheter assembly 2 can be quickly assembled and disassembled. Specifically, the detachable connection of the handle 1 and the catheter assembly 2 can be realized through the slot connection of the housings of the handle 1 and the catheter assembly 2. Further, six degrees of freedom of the handle 1 and the catheter assembly 2 can be limited by the threaded connection, the snap connection, the pin connection and the like, which is more beneficial to maintain the overall stability of the handle. Since the handle 1 is detachably connected to the catheter assembly 2, the safety risk of handle failure can be dealt with quickly, and more trauma to the patient caused by the change from the interventional surgery to the surgical operation due to the handle problem can be avoided. On the other hand, the handle can be reused, which can reduce the cost of each operation and reduce the economic burden of the patient.
2) The present application skillfully utilizes the feature that the outer tube assembly and the inner tube assembly can have relative axial displacement, and combines the detachable connection between the driving member and the outer tube assembly or the inner tube assembly in the third direction, realizing that the handle can be detached from the catheter assembly after the occurrence of the displacement between the handle and the catheter assembly in the first direction. When it is required to connect the handle and the catheter assembly, it is only necessary to align the driving member and the connecting components of the outer tube assembly or the inner tube assembly. That is, the first groove 1111 is engaged with the second groove 2091, and then the two housings are connected by the engaging grooves. During this process, the driving member is connected to the outer tube assembly or the inner tube assembly, and the inner tube assembly or the outer tube assembly is relatively moved in the axial direction during the movement of the housing, which in turn meets the requirements of the relative movement of the housings. In this way, accurate alignment of the handle and the catheter assembly can be achieved, and the connecting process is simple and time-saving.
3) The structural arrangement of the catheter assembly 2 is centralized. That is, the components in contact with blood, such as the outer tube assembly, the inner tube assembly, the evacuation tubes, are all centrally arranged in the catheter assembly 2. The handle 1 and the catheter assembly 2 are individually assembled into an integral assembly, which are independent of each other. Therefore, the manual and electric operations of the handle 1 and the evacuating operation of the catheter assembly 2 do not affect each other. The manually-operated rotating members, the electrically-operated buttons and the evacuation tubes of the catheter assembly are far apart in space, and have no moving contact and will not interfere with each other's operations. Moreover, the evacuation tubes are located in the catheter assembly 2 instead of the handle 1, so there is no need to consider the sealing and leakage problems caused by detaching the handle 1, the structure is simple and reliable, and the handle 1 can be safely reused.
4) The distal end of the catheter assembly 2 is not obstructed by other parts in the handle (such as rotating members or other parts in the handle), leaving more operating space for functional expansion such as catheter closure after the implant is released and rapid retraction of the inner tube.
5) Whether the handle 1 is electric, manual or hybrid of electric and manual, the handle 1 can be manufactured to be detachable from the catheter assembly 2, and has a wide range of adaptability.

## Claims

1. A handle (1) for delivering implants (3), comprising:
a handle housing (10) provided with a hollow first slot (114), and wherein a first connector (109) is provided on the handle housing (10), and the first connector comprises a first engaging component (118) configured to be detachably connected to a catheter assembly(2);
a driving device, and
a transmission component;
wherein the driving device is connected to the transmission component, and controls the transmission component to move; and
the transmission component is provided in the handle housing (10), and comprises a driving member (111); the driving member (111) is moved towards a proximal end or distal end of the handle (1) in the first slot (114) under a control of the driving device;
**characterized in that**
the first engaging component (118) comprises a first engaging slot (1023) and a first engaging strip (1021); and the first engaging strip (1021) protrudes in a second direction and is arranged in the first engaging slot (1023); and
the first engaging slot (1023) extends in a first direction to form a first guiding rail (1024).

2. The handle (1) according to claim 1, wherein the driving device comprises a switch (103), a power supply (104), a control board (105), and a motor (106); the switch (103) is connected to the power supply (104); the power supply (104) is connected to the control board (105); the control board (105) is connected to the motor (106); the motor (106) comprises an output end (1061); and the control board (105) controls the output end (1061) of the motor (106) to rotate.

3. The handle (1) according to claim 2, wherein the transmission component comprises a first large gear (107), a first pinion gear (108), and a screw rod (110); the first large gear (107) is coaxial with and fixedly connected to the output end (1061) of the motor (106); the first large gear (107) is meshed with the first pinion gear (108); the first pinion gear (108) is coaxial with the screw rod (110), and fixedly connected to one end of the screw rod (110); a fixing component is matched and connected to the screw rod (110); and the driving member (111) is fixed on the fixing component;
or, wherein the transmission component comprises a screw rod (110) and a coupling (120); the screw rod (110) is coaxial with the output end (1061) of the motor (106); one end of the coupling (120) is fixedly connected to the output end (1061) of the motor (106), another end of the coupling (120) is fixedly connected to one end of the screw rod (110); a fixing component is matched and connected to the screw rod (110); and the driving member (111) is fixed on the fixing component.

4. The handle (1) according to claim 3, wherein the driving device comprises a manual rotating member (112) and a gear shaft (113); the transmission component comprises a second large gear (115) and a second pinion gear (116); the manual rotating member (112) comprises a manual control member (1121) and a rotating shaft (1122) that are fixedly connected; the manual control member (1121) is arranged outside the handle housing (10); the rotating shaft (1122) passes through the handle housing (10), and is coaxial with and fixedly connected to the gear shaft (113); the gear shaft (113) is coaxial with and fixedly connected to the second large gear (115); the second large gear (115) is meshed with the second pinion gear (116); and the second pinion gear (116) is coaxial with the screw rod (110); and the second pinion gear (116) is fixedly connected to another end of the screw rod (110).

5. The handle (1) according to claim 1, wherein the driving device comprises a manual rotating member (112) and a gear shaft (113); the manual rotating member (112) comprises a manual control member (1121) and a rotating shaft (1122) that are fixedly connected; the manual control member (1121) is arranged outside the handle housing (10); the rotating shaft (1122) passes through the handle housing (10), and is coaxial with and fixedly connected to the gear shaft (113);
preferably, wherein the transmission component comprises a second large gear (115), a second pinion gear (116), and a screw rod (110); the second large gear (115) is coaxial with and fixedly connected to the gear shaft (113); the second large gear (115) is meshed with the second pinion gear (116); the second pinion gear (116) is coaxial with the screw rod (110), and is fixedly connected to an end of the screw rod (110); a fixing component is matched and connected to the screw rod (110); and the driving member (111) is fixed on the fixing component.

6. The handle (1) according to claim 1, wherein the driving device comprises a switch (103), a power supply (104), a control board (105), and a motor (106); the switch (103) is connected to the power supply (104); the power supply (104) is connected to the control board (105); the control board (105) is connected to the motor (106); the motor (106) has a motor shaft; and the control board (105) controls the motor shaft to reciprocate in an axial direction;
preferably, wherein the driving member (111) is fixed on the motor shaft.

7. The handle (1) according to claim 1, wherein the first engaging component (118) comprises a third protruding portion and a fourth protruding portion that are connected to each other; the third protruding portion extends in a first direction, and the fourth protruding portion extends in a third direction; a second opening is formed between the third protruding portion, the fourth protruding portion and an edge of the handle housing (10); and the second opening faces the distal end of the handle (1).

8. The handle (1) according to claim 7, wherein a fastening component (119) is provided on a side of the handle housing (10);
preferably, wherein the fastening component (119) is a threaded knob; the threaded knob comprises a threaded portion (1192) and a knob portion (1191); the knob portion (1191) is provided at an outer side of a distal end of the handle housing (10), and extends out of an edge of the handle housing (10) in a third direction; the threaded portion (1192) passes through the handle housing (10) and is threadedly connected to a fixing member (123) provided in the handle housing (10).

9. The handle (1) according to claim 1, wherein the driving device comprises a manual rotating member (112) and a gear shaft (113); the transmission component comprises a large gear (122) and a pinion gear (121); the manual rotating member (112) comprises a manual control member (1121) and a rotating shaft (1122) that are fixedly connected; the manual control member (1121) is arranged at an outer side of a proximal end of the handle housing (10); the rotating shaft (1122) passes through the handle housing (10), and is coaxial with and fixedly connected to the gear shaft (113); the gear shaft (113) is coaxial and fixedly connected to the large gear (122); the large gear (122) is meshed with the pinion gear (121); and the pinion gear (121) is coaxial with the screw rod (110), and the pinion gear (121) is fixedly connected to another end of the screw rod (110).

10. A catheter assembly (2) for delivering implants (3), comprising:
a catheter assembly housing (20), a second connector being provided on the catheter assembly housing (20); the second connector being configured to be detachably connected to the handle (1) according to any one of claims 1 to 9; the catheter assembly housing (20) being provided with a hollow second slot (211);
an inner tube (2063);
an outer tube (205); and
an outer tube base (209);
wherein the inner tube (2063) passes through the outer tube base (209) and is located in the outer tube (205); the outer tube base (209) is movably sleeved outside the inner tube (2063); and a distal end of the outer tube base (209) is fixedly connected to a proximal end of the outer tube (205);
**characterized in that**:
the second connector comprises a second engaging component (2012) provided on the catheter assembly housing (20) in a first direction; and the outer tube base (209) is moved towards a proximal end or distal end of the catheter assembly (2) in the second slot (211); and
the second engaging component (2012) comprises a first protruding portion (2012a) and a second protruding portion (2012b) that are connected to each other; the first protruding portion (2012a) extends in the first direction, and the second protruding portion (2012b) extends in a third direction; a first opening (2012c) is formed between the first protruding portion (2012a), the second protruding portion (2012b) and an edge of the catheter assembly housing (20); and the first opening (2012c) faces the proximal end of the catheter assembly (2).

11. The catheter assembly (2) according to claim 10, further comprising an inner tube evacuation tube (210); wherein a proximal end of the inner tube evacuation tube (210) is provided on a housing at a proximal end of the catheter assembly housing (20); and a distal end of the inner tube evacuation tube (210) is fixedly connected to a proximal end of the inner tube (2063);
or, wherein an outer tube evacuation tube (208) communicating with the outer tube (205) is provided on the outer tube base (209); and the outer tube evacuation tube (208) extends out of the catheter assembly housing (20).

12. The catheter assembly (2) according to any one of claims 10 to 11, further comprising a stabilizing tube base (203) and a stabilizing tube (204), wherein the stabilizing tube base (203) is fixed on a distal end of the catheter assembly housing (20); a proximal end of the stabilizing tube (204) is fixedly connected to a distal end of the stabilizing tube base (203); a distal end of the outer tube (205) passes through the stabilizing tube base (203) and the stabilizing tube (204);
preferably, wherein a stabilizing tube evacuation tube (207) communicating with the stabilizing tube (204) is provided on the stabilizing tube base (203); and the stabilizing tube evacuation tube (207) extends out of and is exposed to the catheter assembly housing (20).

13. The catheter assembly (2) according to claim 10, wherein the second engaging component (2012) comprises a second engaging slot and a second engaging strip; the second engaging strip protrudes in a second direction and is arranged in the second engaging slot;
preferably, wherein the second engaging slot extends in the first direction to form a second guiding rail.

14. A delivering system for delivering implants (3), comprising:
the handle (1) according to any one of claims 1 to 9; and
the catheter assembly (2) according to any one of claims 10 to 13;
wherein the second connector is detachably connected to the first connector (109); and a position and a shape of the second slot (211) correspond to a position and a shape of the first slot (114) on the handle housing (10);
the outer tube base (209) is fixedly connected to the driving member (111) in an axial direction, and the outer tube base (209) is detachably connected to the driving member (111) in a third direction.

15. The delivering system according to claim 14, wherein when the delivering system comprises the handle (1) according to any one of claims 1 to 9 and the catheter assembly (2) according to any one of claims 10 to 13, a position and a shape of the first engaging component (118) correspond to a position and a shape of the second engaging component (2012).

16. The delivering system according to claim 14 or 15, wherein a first groove (1111) is provided on the driving member (111), a second groove (2091) is provided on the outer tube base (209); and the first groove (1111) cooperates with the second groove (2091) and is sleeved inside the second groove (2091).

17. The delivering system according to claim 14, wherein the first connector (109) has a detaching button (1092) and a third slot (1091); the second connector is a protrusion (2011) having a limiting component in a third direction; a first through hole (1011) and a second through hole (1012) are provided on the handle housing (10); a proximal end of the first connector (109) passes through the first through hole (1011) so that the third slot (1091) is located at an inner side of the handle housing (10), and the detaching button (1092) is located at an outer side of the handle housing (10); the handle housing (10) has a locking position and a detaching position; the detaching button (1092) is capable of being toggled between the locking position and the detaching position; after passing through the second through hole (1012), the protrusion (2011) is located at the inner side of the handle housing (10); when the protrusion (2011) toggles the detaching button (1092) to the locking position, the third slot (1091) is engaged with the protrusion (2011).

18. The delivering system according to claim 15, wherein the first engaging component (118) comprises a first engaging slot (1023) and a first engaging strip (1021); the first engaging strip (1021) protrudes in a second direction and is arranged in the first engaging slot (1023); the second engaging component (2012) comprises a first protruding portion (2012a) and a second protruding portion (2012b) that are connected to each other; a first opening (2012c) is formed between the first protruding portion (2012a), the second protruding portion (2012b) and an edge of the catheter assembly housing (20); and the first opening (2012c) faces a proximal end of the catheter assembly (2) and is engaged with the first engaging strip (1021);
or, wherein a threaded knob is provided on a side of the handle housing (10); the threaded knob comprises a threaded portion (1192) and a knob portion (1191); the knob portion (1191) is provided at an outer side of a distal end of the handle housing (10); and a surface of a proximal end of the knob portion (1191) extends out of an edge of the handle housing (10) in a third direction, to be abutted against an outer side of a distal end of the catheter assembly housing (20).

## Patentansprüche

1. Griff (1) zum Einsetzen von Implantaten (3), umfassend:
ein Griffgehäuse (10), das mit einem ersten Hohlschlitz (114) bereitgestellt ist, und wobei am Griffgehäuse (10) ein erster Verbinder (109) bereitgestellt ist und der erste Verbinder eine erste Eingriffskomponente (118) umfasst, die konfiguriert ist, um lösbar mit einer Katheteranordnung (2) verbunden zu werden;
eine Antriebsvorrichtung, und
eine Getriebekomponente;
wobei die Antriebsvorrichtung mit der Getriebekomponente verbunden ist und die Bewegung der Getriebekomponente steuert; und
die Getriebekomponente im Griffgehäuse (10) bereitgestellt ist und ein Antriebselement (111) umfasst; das Antriebselement (111) unter einer Steuerung der Antriebsvorrichtung in Richtung eines proximalen Endes oder distalen Endes des Griffs (1) im ersten Schlitz (114) bewegt wird;
**dadurch gekennzeichnet, dass**
die erste Eingriffskomponente (118) einen ersten Eingriffsschlitz (1023) und einen ersten Eingriffsstreifen (1021) umfasst; und der erste Eingriffsstreifen (1021) in eine zweite Richtung vorsteht und im ersten Eingriffsschlitz (1023) angeordnet ist; und
der erste Eingriffsschlitz (1023) sich in einer ersten Richtung erstreckt und eine erste Führungsschiene (1024) bildet.

2. Griff (1) nach Anspruch 1, wobei die Antriebsvorrichtung einen Schalter (103), eine Stromversorgung (104), eine Steuereinheit (105) und einen Motor (106) umfasst; der Schalter (103) mit der Stromversorgung (104) verbunden ist; die Stromversorgung (104) mit der Steuereinheit (105) verbunden ist; die Steuereinheit (105) mit dem Motor (106) verbunden ist; der Motor (106) ein Abtriebsende (1061) umfasst; und die Steuereinheit (105) die Drehung des Abtriebsendes (1061) des Motors (106) steuert.

3. Griff (1) nach Anspruch 2, wobei die Getriebekomponente ein erstes großes Zahnrad (107), ein erstes Ritzel (108) und eine Gewindestange (110) umfasst; das erste große Zahnrad (107) koaxial zum Abtriebsende (1061) des Motors (106) und fest mit diesem verbunden ist; das erste große Zahnrad (107) in das erste Ritzel (108) eingreift; das erste Ritzel (108) koaxial zur Gewindestange (110) und fest mit einem Ende der Gewindestange (110) verbunden ist; eine Befestigungskomponente an die Gewindestange (110) angepasst und mit dieser verbunden ist; und das Antriebselement (111) an der Befestigungskomponente befestigt ist;
oder, wobei die Getriebekomponente eine Gewindestange (110) und eine Kupplung (120) umfasst; die Gewindestange (110) koaxial zum Abtriebsende (1061) des Motors (106) ist; ein Ende der Kupplung (120) fest mit dem Abtriebsende (1061) des Motors (106) verbunden ist, ein anderes Ende der Kupplung (120) fest mit einem Ende der Gewindestange (110) verbunden ist; eine Befestigungskomponente an die Gewindestange (110) angepasst und mit dieser verbunden ist; und das Antriebselement (111) an der Befestigungskomponente befestigt ist.

4. Griff (1) nach Anspruch 3, wobei die Antriebsvorrichtung ein manuelles Drehelement (112) und eine Getriebewelle (113) umfasst; die Getriebekomponente ein zweites großes Zahnrad (115) und ein zweites Ritzel (116) umfasst; das manuelle Drehelement (112) ein manuelles Steuerelement (1121) und eine Drehwelle (1122) umfasst, die fest miteinander verbunden sind; das manuelle Steuerelement (1121) außerhalb des Griffgehäuses (10) angeordnet ist; die Drehwelle (1122) durch das Griffgehäuse (10) verläuft und koaxial zur Getriebewelle (113) und fest mit dieser verbunden ist; die Getriebewelle (113) koaxial zum zweiten großen Zahnrad (115) und fest mit diesem verbunden ist; das zweite große Zahnrad (115) in das zweite Ritzel (116) eingreift; und das zweite Ritzel (116) koaxial zur Gewindestange (110) ist; und das zweite Ritzel (116) fest mit einem anderen Ende der Gewindestange (110) verbunden ist.

5. Griff (1) nach Anspruch 1, wobei die Antriebsvorrichtung ein manuelles Drehelement (112) und eine Getriebewelle (113) umfasst; das manuelle Drehelement (112) ein manuelles Steuerelement (1121) und eine Drehwelle (1122) umfasst, die fest miteinander verbunden sind; das manuelle Steuerelement (1121) außerhalb des Griffgehäuses (10) angeordnet ist; die Drehwelle (1122) durch das Griffgehäuse (10) verläuft und koaxial zur Getriebewelle (113) und fest mit dieser verbunden ist;
wobei vorzugsweise die Getriebekomponente ein zweites großes Zahnrad (115), ein zweites Ritzel (116) und eine Gewindestange (110) umfasst; das zweite große Zahnrad (115) koaxial zur Getriebewelle (113) und fest mit dieser verbunden ist; das zweite große Zahnrad (115) in das zweite Ritzel (116) eingreift; das zweite Ritzel (116) koaxial zur Gewindestange (110) und fest mit einem Ende der Gewindestange (110) verbunden ist; eine Befestigungskomponente an die Gewindestange (110) angepasst und mit dieser verbunden ist; und das Antriebselement (111) an der Befestigungskomponente befestigt ist.

6. Griff (1) nach Anspruch 1, wobei die Antriebsvorrichtung einen Schalter (103), eine Stromversorgung (104), eine Steuereinheit (105) und einen Motor (106) umfasst; der Schalter (103) mit der Stromversorgung (104) verbunden ist; die Stromversorgung (104) mit der Steuereinheit (105) verbunden ist; die Steuereinheit (105) mit dem Motor (106) verbunden ist; der Motor (106) eine Motorwelle aufweist; und die Steuereinheit (105) eine Hin- und Herbewegung der Motorwelle in einer Axialrichtung steuert;
wobei das Antriebselement (111) vorzugsweise auf der Motorwelle befestigt ist.

7. Griff (1) nach Anspruch 1, wobei die erste Eingriffskomponente (118) einen dritten vorstehenden Abschnitt und einen vierten vorstehenden Abschnitt umfasst, die miteinander verbunden sind; der dritte vorstehende Abschnitt sich in eine erste Richtung erstreckt und der vierte vorstehende Abschnitt sich in eine dritte Richtung erstreckt; zwischen dem dritten vorstehenden Abschnitt, dem vierten vorstehenden Abschnitt und einer Kante des Griffgehäuses (10) eine zweite Öffnung gebildet ist; und die zweite Öffnung dem distalen Ende des Griffs (1) zugewandt ist.

8. Griff (1) nach Anspruch 7, wobei an einer Seite des Griffgehäuses (10) eine Befestigungskomponente (119) bereitgestellt ist;
wobei vorzugsweise die Befestigungskomponente (119) ein Gewindeknopf ist; der Gewindeknopf einen Gewindeabschnitt (1192) und einen Knopfabschnitt (1191) umfasst; der Knopfabschnitt (1191) an einer Außenseite eines distalen Endes des Griffgehäuses (10) bereitgestellt ist und in einer dritten Richtung aus einer Kante des Griffgehäuses (10) vorsteht; der Gewindeabschnitt (1192) durch das Griffgehäuse (10) verläuft und mit einem im Griffgehäuse (10) bereitgestellten Befestigungselement (123) verschraubt ist.

9. Griff (1) nach Anspruch 1, wobei die Antriebsvorrichtung ein manuelles Drehelement (112) und eine Getriebewelle (113) umfasst; die Getriebekomponente ein großes Zahnrad (122) und ein Ritzel (121) umfasst; das manuelle Drehelement (112) ein manuelles Steuerelement (1121) und eine Drehwelle (1122) umfasst, die fest miteinander verbunden sind; das manuelle Steuerelement (1121) an einer Außenseite eines proximalen Endes des Griffgehäuses (10) angeordnet ist; die Drehwelle (1122) durch das Griffgehäuse (10) verläuft und koaxial zur Getriebewelle (113) und fest mit dieser verbunden ist; die Getriebewelle (113) koaxial zum großen Zahnrad (122) und fest mit diesem verbunden ist; das große Zahnrad (122) in das Ritzel (121) eingreift; und das Ritzel (121) koaxial zur Gewindestange (110) ist und das Ritzel (121) fest mit einem anderen Ende der Gewindestange (110) verbunden ist.

10. Katheteranordnung (2) zum Einsetzen von Implantaten (3), umfassend:
ein Katheteranordnungsgehäuse (20), einen zweiten Verbinder, der am Katheteranordnungsgehäuse (20) bereitgestellt ist; wobei der zweite Verbinder konfiguriert ist, um lösbar mit dem Griff (1) nach einem der Ansprüche 1 bis 9 verbunden zu werden, wobei das Katheteranordnungsgehäuse (20) mit einem zweiten Hohlschlitz (211) bereitgestellt ist;
einen inneren Schlauch (2063);
einen äußeren Schlauch (205); und
einen Sockel (209) des äußeren Schlauchs;
wobei der innere Schlauch (2063) durch den Sockel (209) des äußeren Schlauchs verläuft und sich im äußeren Schlauch (205) befindet; der Sockel (209) des äußeren Schlauchs beweglich außerhalb des inneren Schlauchs (2063) aufgeschoben ist; und ein distales Ende des Sockels (209) des äußeren Schlauchs fest mit einem proximalen Ende des äußeren Schlauchs (205) verbunden ist;
**dadurch gekennzeichnet, dass**:
der zweite Verbinder eine zweite Eingriffskomponente (2012) umfasst, die in einer ersten Richtung am Katheteranordnungsgehäuse (20) bereitgestellt ist; und der Sockel (209) des äußeren Schlauchs im zweiten Schlitz (211) in Richtung eines proximalen Endes oder distalen Endes der Katheteranordnung (2) bewegt wird; und
die zweite Eingriffskomponente (2012) einen ersten vorstehenden Abschnitt (2012a) und einen zweiten vorstehenden Abschnitt (2012b) umfasst, die miteinander verbunden sind; der erste vorstehende Abschnitt (2012a) sich in die erste Richtung erstreckt und der zweite vorstehende Abschnitt (2012b) sich in eine dritte Richtung erstreckt; zwischen dem ersten vorstehenden Abschnitt (2012a), dem zweiten vorstehenden Abschnitt (2012b) und einer Kante des Katheteranordnungsgehäuses (20) eine erste Öffnung (2012c) gebildet ist; und die erste Öffnung (2012c) dem proximalen Ende der Katheteranordnung (2) zugewandt ist.

11. Katheteranordnung (2) nach Anspruch 10, weiter umfassend einen Absaugschlauch (210) des inneren Schlauchs; wobei ein proximales Ende des Absaugschlauchs (210) des inneren Schlauchs an einem Gehäuse an einem proximalen Ende des Katheteranordnungsgehäuses (20) bereitgestellt ist; und ein distales Ende des Absaugschlauchs (210) des inneren Schlauchs fest mit einem proximalen Ende des inneren Schlauchs (2063) verbunden ist;
oder wobei ein mit dem äußeren Schlauch (205) in Verbindung stehender Absaugschlauch (208) des äußeren Schlauchs am Sockel (209) des äußeren Schlauchs bereitgestellt ist und der Absaugschlauch (208) des äußeren Schlauchs aus dem Gehäuse (20) der Katheteranordnung vorsteht.

12. Katheteranordnung (2) nach einem der Ansprüche 10 bis 11, weiter umfassend einen Sockel (203) des Stabilisierungsschlauchs und einen Stabilisierungsschlauch (204), wobei der Sockel (203) des Stabilisierungsschlauchs an einem distalen Ende des Katheteranordnungsgehäuses (20) befestigt ist; ein proximales Ende des Stabilisierungsschlauchs (204) fest mit einem distalen Ende des Sockels (203) des Stabilisierungsschlauchs verbunden ist; ein distales Ende des äußeren Schlauchs (205) durch den Sockel (203) des Stabilisierungsschlauchs und den Stabilisierungsschlauch (204) verläuft;
wobei vorzugsweise ein Absaugschlauch (207) des Stabilisierungsschlauchs, der mit dem Stabilisierungsschlauch (204) in Verbindung steht, auf dem Sockel (203) des Stabilisierungsschlauchs bereitgestellt ist; und der Absaugschlauch (207) des Stabilisierungsschlauchs aus dem Katheteranordnungsgehäuse (20) vorsteht und diesem gegenüber freiliegt.

13. Katheteranordnung (2) nach Anspruch 10, wobei die zweite Eingriffskomponente (2012) einen zweiten Eingriffsschlitz und einen zweiten Eingriffsstreifen umfasst; der zweite Eingriffsstreifen in eine zweite Richtung vorsteht und im zweiten Eingriffsschlitz angeordnet ist;
wobei sich der zweite Eingriffsschlitz vorzugsweise in die erste Richtung erstreckt und eine zweite Führungsschiene bildet.

14. Einsatzsystem zum Einsetzen von Implantaten (3), umfassend:
den Griff (1) nach einem der Ansprüche 1 bis 9; und
die Katheteranordnung (2) nach einem der Ansprüche 10 bis 13;
wobei der zweite Verbinder lösbar mit dem ersten Verbinder (109) verbunden ist;
und eine Position und eine Form des zweiten Schlitzes (211) einer Position und einer Form des ersten Schlitzes (114) am Griffgehäuse (10) entsprechen;
der Sockel (209) des äußeren Schlauchs in einer Axialrichtung fest mit dem Antriebselement (111) verbunden ist und der Sockel (209) des äußeren Schlauchs in einer dritten Richtung lösbar mit dem Antriebselement (111) verbunden ist.

15. Einsatzsystem nach Anspruch 14, wobei, wenn das Einsatzsystem den Griff (1) nach einem der Ansprüche 1 bis 9 und die Katheteranordnung (2) nach einem der Ansprüche 10 bis 13 umfasst, eine Position und eine Form der ersten Eingriffskomponente (118) einer Position und einer Form der zweiten Eingriffskomponente (2012) entsprechen.

16. Einsatzsystem nach Anspruch 14 oder 15, wobei am Antriebselement (111) eine erste Nut (1111) bereitgestellt ist, am Sockel (209) des äußeren Schlauchs eine zweite Nut (2091) bereitgestellt ist und die erste Nut (1111) mit der zweiten Nut (2091) zusammenwirkt und in die zweite Nut (2091) eingeschoben ist.

17. Einsatzsystem nach Anspruch 14, wobei
der erste Verbinder (109) einen Trennknopf (1092) und
einen dritten Schlitz (1091) aufweist; der zweite Verbinder ein Vorsprung (2011) ist, der eine Begrenzungskomponente in einer dritten Richtung aufweist; ein erstes Durchgangsloch (1011) und ein zweites Durchgangsloch (1012) am Griffgehäuse (10) bereitgestellt sind; ein proximales Ende des ersten Verbinders (109) durch das erste Durchgangsloch (1011) verläuft, so dass sich der dritte Schlitz (1091) an einer Innenseite des Griffgehäuses (10) befindet und der Trennknopf (1092) sich an einer Außenseite des Griffgehäuses (10) befindet; das Griffgehäuse (10) eine Verriegelungsposition und eine Trennposition aufweist; der Trennknopf (1092) zwischen der Verriegelungsposition und der Trennposition umgeschaltet werden kann; nach dem Durchdringen des zweiten Durchgangslochs (1012) der Vorsprung (2011) sich an der Innenseite des Griffgehäuses (10) befindet; wenn der Vorsprung (2011) den Trennknopf (1092) in die Verriegelungsposition bringt, greift der dritte Schlitz (1091) in den Vorsprung (2011) ein.

18. Einsatzsystem nach Anspruch 15, wobei die erste Eingriffskomponente (118) einen ersten Eingriffsschlitz (1023) und einen ersten Eingriffsstreifen (1021) umfasst; der erste Eingriffsstreifen (1021) in eine zweite Richtung vorsteht und im ersten Eingriffsschlitz (1023) angeordnet ist; die zweite Eingriffskomponente (2012) einen ersten vorstehenden Abschnitt (2012a) und einen zweiten vorstehenden Abschnitt (2012b) umfasst, die miteinander verbunden sind; eine erste Öffnung (2012c) zwischen dem ersten vorstehenden Abschnitt (2012a), dem zweiten vorstehenden Abschnitt (2012b) und einer Kante des Katheteranordnungsgehäuses (20) gebildet ist; und die erste Öffnung (2012c) einem proximalen Ende der Katheteranordnung (2) zugewandt und mit dem ersten Eingriffsstreifen (1021) in Eingriff ist;
oder wobei an einer Seite des Griffgehäuses (10) ein Gewindeknopf bereitgestellt ist; der Gewindeknopf einen Gewindeabschnitt (1192) und einen Knopfabschnitt (1191) umfasst; der Knopfabschnitt (1191) an einer Außenseite eines distalen Endes des Griffgehäuses (10) bereitgestellt ist; und eine Oberfläche eines proximalen Endes des Knopfabschnitts (1191) in einer dritten Richtung aus einer Kante des Griffgehäuses (10) vorsteht, um an einer Außenseite eines distalen Endes des Katheteranordnungsgehäuses (20) anzuliegen.

## Revendications

1. Poignée (1) pour la mise en place d'implants (3), comprenant :
un boîtier de poignée (10) muni d'une première fente creuse (114), et dans lequel un premier connecteur (109) est prévu sur le boîtier de poignée (10), et le premier connecteur comprend un premier composant de prise (118) configuré pour être connecté de manière détachable à un ensemble cathéter (2) ;
un dispositif de commande, et
un composant de transmission ;
dans laquelle le dispositif de commande est relié au composant de transmission et commande le déplacement de ce dernier ; et
le composant de transmission est ménagé dans le boîtier de poignée (10) et comprend un élément de commande (111) ; l'élément de commande (111) est déplacé vers une extrémité proximale ou distale de la poignée (1) dans la première fente (114) sous le contrôle du dispositif de commande ;
**caractérisé en ce que**
le premier élément de prise (118) comprend une première fente de prise (1023) et une première bande de prise (1021) ; et la première bande de prise (1021) fait saillie dans une deuxième direction et est disposée dans la première fente de prise (1023) ; et
la première fente de prise (1023) s'étend dans une première direction pour former un premier rail de guidage (1024).

2. Poignée (1) selon la revendication 1, dans laquelle le dispositif de commande comprend un interrupteur (103), une alimentation (104), une carte de commande (105) et un moteur (106) ; l'interrupteur (103) est connecté à l'alimentation (104) ; l'alimentation (104) est connectée à la carte de commande (105) ; la carte de commande (105) est connectée au moteur (106) ; le moteur (106) comprend une extrémité de sortie (1061) ; et la carte de commande (105) commande la rotation de l'extrémité de sortie (1061) du moteur (106).

3. Poignée (1) selon la revendication 2, dans laquelle l'élément de transmission comprend un premier grand engrenage (107), un premier pignon (108) et une tige filetée (110) ; le premier grand engrenage (107) est coaxial à l'extrémité de sortie (1061) du moteur (106) et fixé à celle-ci ; le premier grand engrenage (107) est en prise avec le premier pignon (108) ; le premier pignon (108) est coaxial à la tige filetée (110) et fixé à l'une des extrémités de la tige filetée (110) ; un élément de fixation est adapté et fixé à la tige filetée (110) ; et l'élément de commande (111) est fixé sur l'élément de fixation ;
ou, dans laquelle l'élément de transmission comprend une tige filetée (110) et un accouplement (120) ; la tige filetée (110) est coaxiale avec l'extrémité de sortie (1061) du moteur (106) ; une extrémité de l'accouplement (120) est fixée à l'extrémité de sortie (1061) du moteur (106), l'autre extrémité de l'accouplement (120) est fixée à une extrémité de la tige filetée (110) ; un élément de fixation est adapté et connecté à la tige filetée (110) ; et l'élément de commande (111) est fixé sur l'élément de fixation.

4. Poignée (1) selon la revendication 3, dans laquelle le dispositif de commande comprend un élément rotatif manuel (112) et un arbre d'engrenage (113) ; l'élément de transmission comprend un deuxième grand engrenage (115) et un deuxième pignon (116) ; l'élément rotatif manuel (112) comprend un élément de commande manuelle (1121) et un arbre de rotation (1122) reliés entre eux ; l'élément de commande manuelle (1121) est disposé à l'extérieur du boîtier de la poignée (10) ; l'arbre de rotation (1122) traverse le boîtier de la poignée (10) et est coaxial à l'arbre d'engrenage (113) auquel il est relié ; l'arbre d'engrenage (113) est coaxial au deuxième grand engrenage (115) auquel il est relié ; le deuxième grand engrenage (115) est en prise avec le deuxième pignon (116) ; et le deuxième pignon (116) est coaxial à la tige filetée (110) et le deuxième pignon (116) est fixé à une autre extrémité de la tige filetée (110).

5. Poignée (1) selon la revendication 1, dans laquelle le dispositif de commande comprend un élément rotatif manuel (112) et un arbre d'engrenage (113) ; l'élément rotatif manuel (112) comprend un élément de commande manuelle (1121) et un arbre de rotation (1122) qui sont reliés de manière fixe ; l'élément de commande manuelle (1121) est disposé à l'extérieur du boîtier de la poignée (10) ; l'arbre de rotation (1122) traverse le boîtier de la poignée (10) et est coaxial avec l'arbre d'engrenage (113) auquel il est relié de manière fixe ;
de préférence, dans laquelle le composant de transmission comprend un deuxième grand engrenage (115), un deuxième pignon (116) et une tige filetée (110) ; le deuxième grand engrenage (115) est coaxial avec et fixé à l'arbre d'engrenage (113) ; le deuxième grand engrenage (115) est en prise avec le deuxième pignon (116) ; le deuxième pignon (116) est coaxial avec la tige filetée (110) et est fixé à une extrémité de la tige filetée (110) ; un élément de fixation est adapté et connecté à la tige filetée (110) ; et l'élément de commande (111) est fixé sur l'élément de fixation.

6. Poignée (1) selon la revendication 1, dans laquelle le dispositif de commande comprend un interrupteur (103), une alimentation électrique (104), une carte de commande (105) et un moteur (106) ; l'interrupteur (103) est connecté à l'alimentation électrique (104) ; l'alimentation électrique (104) est connectée à la carte de commande (105) ; la carte de commande (105) est connectée au moteur (106) ; le moteur (106) possède un arbre moteur ; et la carte de commande (105) commande l'arbre moteur pour qu'il effectue un mouvement de va-et-vient axial ;
de préférence, dans laquelle l'élément de commande (111) est fixé sur l'arbre du moteur.

7. Poignée (1) selon la revendication 1, dans laquelle le premier élément de prise (118) comprend une troisième partie saillante et une quatrième partie saillante qui sont reliées entre elles ; la troisième partie saillante s'étend dans une première direction et la quatrième partie saillante s'étend dans une troisième direction ; une deuxième ouverture est formée entre la troisième partie saillante, la quatrième partie saillante et un bord du boîtier de la poignée (10) ; et la deuxième ouverture fait face à l'extrémité distale de la poignée (1).

8. Poignée (1) selon la revendication 7, dans laquelle un élément de fixation (119) est prévu sur un côté du boîtier de la poignée (10) ;
de préférence, dans laquelle l'élément de fixation (119) est un bouton fileté ; le bouton fileté comprend une partie filetée (1192) et une partie bouton (1191) ; la partie bouton (1191) est située du côté extérieur d'une extrémité distale du boîtier de la poignée (10) et s'étend hors d'un bord du boîtier de la poignée (10) dans une troisième direction ; la partie filetée (1192) traverse le boîtier de la poignée (10) et est reliée par filetage à un élément de fixation (123) prévu dans le boîtier de la poignée (10).

9. Poignée (1) selon la revendication 1, dans laquelle le dispositif de commande comprend un élément rotatif manuel (112) et un arbre d'engrenage (113) ; l'élément de transmission comprend un grande engrenage (122) et un pignon (121) ; l'élément rotatif manuel (112) comprend un dispositif de commande manuelle (1121) et un arbre de rotation (1122) reliés entre eux ; le dispositif de commande manuelle (1121) est disposé à l'extérieur de l'extrémité proximale du boîtier de la poignée (10) ; l'arbre de rotation (1122) traverse le boîtier de la poignée (10) et est coaxial à l'arbre d'engrenage (113) auquel il est relié ; l'arbre d'engrenage (113) est coaxial au grand engrenage (122) et relié à celui-ci ; le grand engrenage (122) est engrené avec le pignon (121) ; et le pignon (121) est coaxial avec la tige filetée (110), et le pignon (121) est fixé à une autre extrémité de la tige filetée (110).

10. Ensemble cathéter (2) pour la mise en place d'implants (3), comprenant :
un boîtier d'ensemble cathéter (20), un deuxième connecteur étant prévu sur le boîtier d'ensemble cathéter (20) ; le deuxième connecteur étant configuré pour être connecté de manière amovible à la poignée (1) selon l'une quelconque des revendications 1 à 9 ; le boîtier de l'ensemble cathéter (20) étant muni d'une deuxième fente creuse (211) ;
un tube intérieur (2063) ;
un tube extérieur (205) ; et
une base de tube extérieur (209) ;
dans lequel le tube intérieur (2063) passe à travers la base du tube extérieur (209) et est situé dans le tube extérieur (205) ; la base du tube extérieur (209) est gainée de manière mobile à l'extérieur du tube intérieur (2063) ; et une extrémité distale de la base du tube extérieur (209) est reliée de manière fixe à une extrémité proximale du tube extérieur (205) ;
**caractérisé en ce que** :
le deuxième connecteur comprend un deuxième élément de prise (2012) disposé sur le boîtier de l'ensemble cathéter (20) dans une première direction ; et la base du tube extérieur (209) est déplacée vers une extrémité proximale ou distale de l'ensemble cathéter (2) dans la deuxième fente (211) ; et
le deuxième composant de prise (2012) comprend une première partie saillante (2012a) et une deuxième partie saillante (2012b) reliées entre elles ; la première partie saillante (2012a) s'étend dans la première direction et la deuxième partie saillante (2012b) s'étend dans une troisième direction ; une première ouverture (2012c) est formée entre la première partie saillante (2012a), la deuxième partie saillante (2012b) et un bord du boîtier de l'ensemble cathéter (20) ; et la première ouverture (2012c) fait face à l'extrémité proximale de l'ensemble cathéter (2).

11. Ensemble cathéter (2) selon la revendication 10, comprenant en outre un tube d'évacuation intérieur (210) ; dans lequel une extrémité proximale du tube d'évacuation intérieur (210) est prévue sur un boîtier à une extrémité proximale du boîtier de l'ensemble cathéter (20) ; et une extrémité distale du tube d'évacuation intérieur (210) est connectée de manière fixe à une extrémité proximale du tube intérieur (2063) ;
ou, dans lequel un tube d'évacuation du tube extérieur (208) communiquant avec le tube extérieur (205) est prévu sur la base du tube extérieur (209) ; et le tube d'évacuation du tube extérieur (208) s'étend hors du boîtier de l'ensemble cathéter (20).

12. Ensemble cathéter (2) selon l'une quelconque des revendications 10 à 11, comprenant en outre une base de tube stabilisateur (203) et un tube stabilisateur (204), dans lequel la base de tube stabilisateur (203) est fixée sur une extrémité distale du boîtier de l'ensemble cathéter (20) ; une extrémité proximale du tube stabilisateur (204) est fixée de manière fixe à une extrémité distale de la base de tube stabilisateur (203) ; une extrémité distale du tube extérieur (205) passe à travers la base de tube stabilisateur (203) et le tube stabilisateur (204) ;
de préférence, dans lequel un tube d'évacuation du tube stabilisateur (207) communiquant avec le tube stabilisateur (204) est prévu sur la base du tube stabilisateur (203) ; et le tube d'évacuation du tube stabilisateur (207) s'étend hors du boîtier de l'ensemble cathéter (20) et y est exposé.

13. Ensemble cathéter (2) selon la revendication 10, dans lequel le deuxième composant de prise (2012) comprend une deuxième fente de prise et une deuxième bande de prise ; la deuxième bande de prise fait saillie dans une deuxième direction et est disposée dans la deuxième fente de prise ;
de préférence, dans lequel la deuxième fente de prise s'étend dans la première direction pour former un deuxième rail de guidage.

14. Système de distribution pour la pose d'implants (3), comprenant :
la poignée (1) selon l'une quelconque des revendications 1 à 9 ; et
l'ensemble cathéter (2) selon l'une quelconque des revendications 10 à 13 ;
dans lequel le deuxième connecteur est connecté de manière détachable au premier connecteur (109) ;
et une position et une forme de la deuxième fente (211) correspondent à une position et une forme de la première fente (114) sur le boîtier de la poignée (10) ;
la base du tube extérieur (209) est raccordée de manière fixe à l'élément de commande (111) dans une direction axiale, et la base du tube extérieur (209) est raccordée de manière amovible à l'élément de commande (111) dans une troisième direction.

15. Système de distribution selon la revendication 14, dans lequel lorsque le système de distribution comprend la poignée (1) selon l'une quelconque des revendications 1 à 9 et l'ensemble cathéter (2) selon l'une quelconque des revendications 10 à 13, la position et la forme du premier composant de prise (118) correspondent à la position et à la forme du deuxième composant de prise (2012).

16. Système de distribution selon la revendication 14 ou 15, dans lequel une première rainure (1111) est prévue sur l'élément de commande (111), une deuxième rainure (2091) est prévue sur la base du tube extérieur (209) ; et la première rainure (1111) coopère avec la deuxième rainure (2091) et est logée à l'intérieur de la deuxième rainure (2091).

17. Système de distribution selon la revendication 14, dans lequel
le premier connecteur (109) possède un bouton de détachement (1092) et
une troisième fente (1091) ; le deuxième connecteur est une protubérance (2011) comportant un élément de limitation dans une troisième direction ; un premier trou traversant (1011) et un deuxième trou traversant (1012) sont prévus sur le boîtier de la poignée (10) ; une extrémité proximale du premier connecteur (109) traverse le premier trou traversant (1011) de sorte que la troisième fente (1091) soit située du côté intérieur du boîtier de la poignée (10), et le bouton de détachement (1092) du côté extérieur du boîtier de la poignée (10) ; le boîtier de la poignée (10) possède une position de verrouillage et une position de détachement ; le bouton de détachement (1092) peut être basculé entre la position de verrouillage et la position de détachement ; après avoir traversé le deuxième trou traversant (1012), la protubérance (2011) se trouve du côté intérieur du boîtier de la poignée (10). lorsque la protubérance (2011) bascule le bouton de détachement (1092) en position de verrouillage, la troisième fente (1091) est mise en prise avec la protubérance (2011).

18. Système de distribution selon la revendication 15, dans lequel le premier élément de prise (118) comprend une première fente de prise (1023) et une première bande de prise (1021) ; la première bande de prise (1021) fait saillie dans une deuxième direction et est disposée dans la première fente de prise (1023) ; le deuxième élément de prise (2012) comprend une première partie saillante (2012a) et une deuxième partie saillante (2012b) reliées entre elles ; une première ouverture (2012c) est formée entre la première partie saillante (2012a), la deuxième partie saillante (2012b) et un bord du boîtier de l'ensemble cathéter (20) ; et la première ouverture (2012c) fait face à une extrémité proximale de l'ensemble cathéter (2) et est mise en prise avec la première bande de prise (1021) ;
ou, dans lequel un bouton fileté est prévu sur un côté du boîtier de la poignée (10) ; le bouton fileté comprend une partie filetée (1192) et une partie bouton (1191) ; la partie bouton (1191) est prévue sur le côté extérieur d'une extrémité distale du boîtier de la poignée (10) ; et une surface d'une extrémité proximale de la partie bouton (1191) s'étend hors d'un bord du boîtier de la poignée (10) dans une troisième direction, pour venir en butée contre un côté extérieur d'une extrémité distale du boîtier de l'ensemble cathéter (20).
